(19) Europäisches Patentamt
European Patent Office
Office européen des brevets

(11) EP 1 051 990 B1

# (12) EUROPEAN PATENT SPECIFICATION

(45) Date of publication and mention of the grant of the patent:
**29.10.2008 Bulletin 2008/44**

(21) Application number: **98945628.0**

(22) Date of filing: **05.10.1998**

(51) Int Cl.:
***A61M 25/10*** *(2006.01)* ***A61L 33/00*** *(2006.01)*

(86) International application number:
**PCT/JP1998/004504**

(87) International publication number:
**WO 1999/038557 (05.08.1999 Gazette 1999/31)**

## (54) Balloon catheter and method of production

Ballonkatheter sowie Herstellungsverfahren

Sonde ä ballonnet et procédé de production

(84) Designated Contracting States:
**DE FR GB IT**

(30) Priority: **30.01.1998 JP 1953998**

(43) Date of publication of application:
**15.11.2000 Bulletin 2000/46**

(73) Proprietor: **KANEKA CORPORATION**
**Osaka-shi, Osaka 530-0005 (JP)**

(72) Inventors:
- **MAEDA, Hiromi**
  **Uji-shi,**
  **Kyoto 611-0011 (JP)**
- **MIKI, Shogo**
  **Suita-shi,**
  **Osaka 565-0824 (JP)**
- **NISHIDE, Takuji**
  **Settsu-shi,**
  **Osaka 566-0072 (JP)**

(74) Representative: **Vossius & Partner**
**Siebertstrasse 4**
**81675 München (DE)**

(56) References cited:

| | |
|---|---|
| **EP-A- 0 669 142** | **EP-A- 0 768 097** |
| **WO-A-95/23619** | **WO-A-96/00099** |
| **WO-A-96/25970** | **WO-A-97/29800** |
| **GB-A- 2 073 219** | **JP-A- 1 270 872** |
| **JP-A- 6 063 120** | **JP-A- 6 125 989** |
| **JP-A- 8 500 760** | **US-A- 4 373 009** |
| **US-A- 4 464 176** | **US-A- 5 470 322** |
| **US-A- 5 645 533** | |

- **PATENT ABSTRACTS OF JAPAN vol. 1999, no. 02, 26 February 1999 (1999-02-26) & JP 10 290837 A (KANEGAFUCHI CHEM IND CO LTD), 4 November 1998 (1998-11-04)**
- **PATENT ABSTRACTS OF JAPAN vol. 1999, no. 03, 31 March 1999 (1999-03-31) & JP 10 314297 A (KANEGAFUCHI CHEM IND CO LTD), 2 December 1998 (1998-12-02)**

EP 1 051 990 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

This invention relates to a balloon catheter and to a method for manufacturing a catheter shaft and balloon used therein, and more particularly relates to a balloon catheter used in percutaneous transluminal angioplasty (PTA) or percutaneous transluminal coronary angioplasty (PTCA), in which constricted areas or obstructions such as in the coronary artery, limb arteries, the renal artery, or peripheral vessels are treated by dilation, and to a method for manufacturing the catheter shaft and balloon of this balloon catheter.

A balloon catheter is primarily made up of a catheter shaft and a vascular dilation balloon provided to the distal end portion of this catheter shaft. At least two lumens (cavities) are formed in the interior of the catheter shaft. One of these is a guide wire lumen through which a guide wire is passed, and the second is an inflation lumen through which a pressure fluid such as a contrast medium or physiological saline is passed in order to inflate or deflate the balloon. Angioplasty using a balloon catheter such as this is conducted by the following procedure. First, a guide catheter is inserted through the femoral artery and positioned at the far end of the inlet to the coronary artery, after which a guide wire that goes through the balloon catheter is advanced past a constricted site in the coronary artery, then the balloon catheter is advanced along the guide wire and inflated when the balloon is located at the constriction. After the constriction has been dilated, the balloon is deflated and taken out of the body. A balloon catheter such as this is not limited to the treatment of arterial constrictions, and can be used in many other medical applications, including insertion into blood vessels and insertion into various bodily cavities.

Some of the characteristics required of a catheter shaft are its pushability, which is a measure of how efficiently the pushing force applied to the proximal end is transmitted to the distal end, the flexibility of the portion 20 to 30 cm from its distal end, that is, its trackability, which allows this portion to proceed smoothly through curved blood vessels, and its diameter, which needs to be small enough to allow insertion into narrow blood vessels. Since the balloon provided at the distal end is inflated or deflated by introducing a pressure fluid into the inflation lumen, the catheter shaft must be strong enough to withstand the pressure from this pressure fluid. The modulus of elasticity of the catheter shaft needs to be raised in order to raise this pressure resistance strength and improve the pushability, while the modulus of elasticity of the catheter shaft needs to be lowered in order to improve trackability. These are mutually conflicting characteristics, so a catheter shaft needs to strike a good balance between these two characteristics.

The balloon catheters in use today can be divided into over-the-wire balloon types and monorail types. An over-the-wire balloon catheter has a structure in which the guide wire lumen extends the entire length of the catheter, from the farthest end to the nearest end of the catheter. Monorail types are discussed in detail in U.S. Patents 4,762,129 and 4,748982, in which the lumen used for guide wire passage is formed only in the distal portion of the catheter, and the guide wire exits the catheter on the proximal side of this distal portion.

The most salient feature of an over-the-wire balloon catheter is that because the guide wire passes through the interior of the catheter over the entire length of the catheter, there is no slackening of the guide wire and good pushability is obtained even if an obstacle is encountered that would hinder the advance of the guide wire at the catheter distal end portion. As a result, a back-up force can be imparted to the guide wire, making it possible for the guide wire to pass more easily through highly constricted afflicted sites. However, in the event that the balloon catheter has to be replaced for a reason such as the inability to obtain the desired inflation diameter, if a guide wire of ordinary length is being used, an extension guide wire will have to be used, so replacing the balloon catheter takes more time and trouble. Relatively long guide wires that do not require the use of an extension guide wire do exist, but when these guide wires are used, it is difficult to control the wire so that it will pass through the afflicted site, and for this reason a guide wire of ordinary length is usually used.

An advantage to a monorail type of balloon catheter in regard to this point is that although the pushability is not as good, there is no need to use an extension guide wire, so the balloon catheter can be replaced more easily and quickly. This shortens the time required to perform PTCA, and allows PTCA to be performed more times per day. Furthermore, a monorail type of balloon catheter is advantageous because the number of catheters needed for treatment can be kept low and the cost of the PTCA kept down, and because cases involving the use of a stent are on the rise in recent years. Specifically, in a case that requires a stent from the outset, such as a case in which dissection of the vascular wall is likely to occur, after a constricted site has been dilated with a monorail type balloon catheter, this catheter is temporarily pulled out, a stent is then placed at the distal end of the same monorail type balloon catheter and quickly delivered to the constricted site, and the stent is disposed at this constricted site. This offers considerable benefits to both physician and patient, so the use of monorail type balloon catheters has been increasing rapidly in recent years.

Examples of various conventional balloon catheters, and the problems encountered with them, will now be discussed.

US 5,470,322 describes a multi-lumen catheter shaft with a stiffness that varies along the length of the shaft in order to provide both pushability and steerability.

WO 96/00099 describes catheters having a disposable distal body having an expansible dilatation balloon and a reusable proximal body releasably joined through a connection, wherein the proximal body is constructed of a rugged,

resterilizable material.

WO 97/29800 describes a multi-section catheter having a proximal section, a distal section axially adjacent to the proximal section and an injection-molded transition section connecting the proximal section to the distal section, and a method for axially joining adjacent catheter body sections.

WO 96/25970 describes a reinforced monorail balloon catheter having a stiffening wire attached to the inner wall of the catheter shaft at a plurality of points along its length in order to provide strength to the proximal portion of the catheter.

In particular, the following (1) to (4) are conventional examples characterized by the structure of the catheter shaft.

(1) Japanese Laid-Open Patent Applications S63-288169 and H5-192410 discuss balloon catheters comprising a dual-lumen shaft. These balloon catheters, however, have a single structure for the lumen tube, including the dual lumen, and merely involve adjustment of the rigidity in the axial direction, so they do not represent the good balance between pushability and trackability that is required today.

(2) Japanese Laid-Open Patent Application H7-132147 discloses a catheter shaft structure in which a plurality of tubes (inflation lumen tube and guide wire lumen tube) each composed of a different resin material each suited to its own function, and a flexible control means such as a core wire are bonded using an adhesive agent or using a heat-shrink tube from the outside.

However, when a catheter shaft with a structure such as this is manufactured using a heat-shrink tube, depending on the conditions under which the heat-shrink tube shrinks, the inflation lumen or guide wire lumen may be flattened or reduced in diameter. Another problem is that it is exceedingly difficult to line up the plurality of tubes, core wire, etc., without any twisting over the entire length of the catheter in the step prior to the shrinkage of the heat-shrink tube. On the other hand, when a catheter shaft with the above structure is manufactured using an adhesive agent, slight changes in the temperature or humidity during manufacture can affect the final hardness and adhesive strength, and this can affect the flexibility and strength of the catheter shaft itself. These problems inevitably result in a dramatic drop in yield and a rise in manufacturing cost.

(3) Japanese Patent 2,505,954 discloses a catheter shaft structure comprising [i] a tube with a specific inside diameter and a specific wall thickness and [ii] a variable-thickness tube (multi-lumen tube) having at least one lumen with a specific inside diameter and being equipped with a wall thickness portion where the wall thickness varies continuously in the peripheral direction, wherein the tube is embedded in the portion where the wall thickness is large.

The first object of the present invention is to enhance the dimensional precision of a multi-lumen tube, and the second object is to eliminate bumpiness and roughness on the inner surface of the guide wire lumen. Accordingly, the invention is characterized in that extrusion molding is performed such that the outside of a tube into which a mandrel has been inserted is covered with a resin that will form a variable-thickness tube. The manufacturing method of the present invention cannot, however, be applied, and the desired structure cannot be obtained, when the melting point of the embedded tube is far lower than the melting point of the multi-lumen tube.

Also, with the present invention, the outer surface of another tube embedded in that portion of the variable-thickness tube where the wall thickness is large is brought into direct contact with the other lumen in order to make the wall thickness more uniform in the axial direction at the interface between the two lumens. Accordingly, when the pressure fluid is introduced into one of the lumens or tubes, the interface portion is apt to be deformed because it is much weaker than the other surrounding portion.

(4) Recent years have seen the frequent use of catheter shafts with a coaxial structure, in which an outer tube is disposed coaxially around an inner tube that forms the guide wire lumen, and the space between the inner tube and outer tube forms the inflation lumen. At the distal end of this catheter shaft, the ends of the balloon are fastened to the end portion of the outer tube and the end portion of the inner tube, which protrudes farther than the outer tube, and at the proximal end of this catheter shaft, the inner tube and outer tube are fastened so that each one communicates with its own manifold port, thereby constituting a balloon catheter. Thus, the inner tube and outer tube are fastened at two places: the proximal end portion of the manifold and the most distal end portion of the catheter shaft, and the inner tube and outer tube are not fastened by any means at all between these two places. Consequently, there is a loss of the above-mentioned pushability, and due to differences in shrinkage in the axial direction between the inner tube and outer tube, so-called rippling tends to occur, in which the balloon shrinks in the axial direction.

The following (5) to (9) are examples of conventional monorail type balloon catheters.

(5) U.S. Patent 4,762,129 discloses a catheter structure in which a guide wire passage tube is provided adjacent to the inflation lumen tube, but only in the proximity of the distal end portion of the catheter. Therefore, the most distal end of the guide wire passage tube forms a guide wire inlet all by itself.

With this catheter structure, however, a large step is produced at the guide wire inlet, and this step can damage blood vessel walls when the balloon catheter is pulled out, and in a worst case, there is the danger that the step will snag on the blood vessel walls or in the guide catheter, making it difficult even to pull out the balloon catheter.

(6) U.S. Patent 4,748,982 discloses a catheter shaft in which the distal shaft and proximal shaft both have a bi-lumen tube (dual-lumen tube) structure. One of the characteristics of this invention is that the guide wire lumen located on the distal portion side of the proximal-side bi-lumen tube is plugged, and the distal portion of the bi-lumen tube is cut to form the guide wire inlet. Also disclosed is a method for joining the distal shaft and the proximal shaft, in which a mandrel is passed through mutually communicating lumens of the two shafts, the shafts are butted up against one another, and a glass mold is used to heat-fuse and join the shafts together. The above-mentioned cutting, however, requires skill on the part of the worker, and the technical sophistication of the worker tends to be reflected in the quality of the finished product, so a high yield cannot be expected. The cost is therefore relatively high, among other problems.

(7) Japanese Laid-Open Patent Application H2-277465 discloses a monorail type balloon catheter in which the catheter shaft comprises a proximal end portion shaft, a middle portion shaft, and a distal end portion shaft. One of the characteristics of this invention is that the middle portion shaft has two lumens: a guide wire passage lumen and an inflation lumen for inflating the balloon, and the distal end portion shaft only has a guide wire passage lumen. The method for joining these shafts is given as fusing them together in a state in which mandrels have been inserted into the mutually communicating lumens of the various shafts. No specific mention is made about the method for forming a guide wire inlet, but judging from the figures and the contents of the Specification, it seems that cutting is performed on the guide wire passage lumen located at the most proximal portion of the middle bi-lumen tube. As mentioned above, this cutting requires skill on the part of the worker, so a high yield cannot be expected. The cost is therefore relatively high, among other problems.

(8) Published Japanese Translation of PCT Application H6-507105 discloses a monorail type balloon catheter equipped with a main shaft portion formed from a metal tube, and a plastic end shaft portion having a balloon at its distal end and connected to this main shaft portion, in order to enhance the vertical strength of the catheter shaft. In this publication it is stated that a crescent-shaped crimp is formed in the tube of the end shaft portion in the proximity of the guide wire inlet using a die mechanism and a molding plate with a crescent-shaped distal end. This tube of the end shaft portion (distal shaft) and tube of the main shaft portion (proximal shaft) are joined with an adhesive agent.

However, joining with an adhesive agent results in variance in the adhesive agent viscosity and in the adhesive strength between lots, and the viscosity of the adhesive agent may vary in the joining step, which means that the bonding clearance between shafts has to be adjusted, a plasma treatment is required, and so forth. Thus, the control and manufacturing steps become more complicated, the yield decreases, and the manufacturing cost is higher.

The above publication states that a crescent-shaped crimp is formed in the tube of the end shaft portion, which changes the cross sectional shape of the inflation lumen of the proximal shaft from circular to crescent-shaped. It is difficult, however, to join an inflation lumen whose cross section has been worked into a crescent shape to the neck of a balloon member. This is because the high-pressure fluid that passes through the inflation lumen tends to leak from this joint, and preventing this leakage results in a more complicated manufacturing process. Furthermore, a step is present at the guide wire inlet described in the above publication, which tends to be a hindrance in moving the balloon catheter forward or backward in a blood vessel.

(9) Published Japanese Translation of PCT Application H6-506124 discloses a monorail type balloon catheter comprising a main shaft portion formed from a high-tensile strength metal in order to prevent breakage at the distal portion and to enhance pushability, and a middle (actually at the distal portion) resin shaft having a coil for preventing breakage. This balloon catheter, just as with the balloon catheter in Published Japanese Translation of PCT Application H6-507105, has a crimp formed so that the inflation lumen is changed from being circular to crescent-shaped, and the inflation lumen is formed in the crimp portion, so the same problems as above are encountered.

The following (10) to (13) are examples of conventional balloons and methods for their manufacture.

(10) Published Japanese Translation of PCT Application H3-63908 (title of the invention: Method for Manufacturing Catheter Balloon from Flexible, High-Molecular Weight, Biaxially-Drawn Polymer) discloses a balloon in which the tensile strength of the balloon walls has been raised by biaxially drawing a material composed of a polyethylene terephthalate homopolyester. However, because a polyethylene terephthalate homopolyester is a material with extremely high crystallinity, a balloon produced using this material is relatively hard. Consequently, pin holes develop in the balloon, and winging (a serious problem in which the wrapped portion spreads out like the wings of a bird in the radial direction) occurs when the balloon is rewrapped after a dilation treatment.

(11) Japanese Laid-Open Patent Application H3-57462 (title of the invention: Balloon for Medical Device, and Molding Thereof) states that it is possible to control the elongation (inflation profile) of a balloon as desired, from compliant to non-compliant, by biaxially drawing a tube composed of a nylon material or polyamide material and varying the radial orientation (draw ratio), and mentions nylon materials and polyamide materials as materials with which this is possible. However, nylon itself is a highly crystalline resin, and therefore if the wall thickness of a balloon produced from nylon exceeds 20 $\mu$m, the above-mentioned problem of winging will be encountered during rewrapping. Also, when a balloon is molded from a nylon material or polyamide material, there is a great deal of variance (standard

deviation) in the bursting pressure thereof, so if the diameter of the balloon is 3.0 mm and the wall thickness is less than 20 μm, the rated bursting pressure defined in FDA guidelines has a 12 atm limit.

(12) Japanese Laid-Open Patent Application H6-304920 (title of the invention: Inflatable Balloon with Elastic Stress Response Inflatability, and Method for Manufacturing Same) discloses a technique for manufacturing a balloon using a "block copolymer having a region of molecular chain interaction separated by a region where individual portions of the polymer chain can unwind." The main object of the invention pertaining to this application is to enhance elastic stress response and tensile strength (to raise the average bursting pressure), and more particularly, to prevent winging from occurring during rewrapping, which is attributable to heat-shrinkage of the balloon, even when this balloon is heated and sterilized at 50 to 60°C. All of the soft segments of the block copolymer materials given as examples in this publication are polyester.

(13) International Patent WO 95/23619 discloses a balloon made from a polyamide- or polyester-based thermoplastic elastomer. These thermoplastic elastomers are characterized in that the hard segment is a polyamide or polyester and the soft segment is a polyether. The object of this invention is to produce a balloon that has high wall tensile strength, thin walls, and characteristics ranging from compliant to semi-compliant by using one of these thermoplastic elastomers.

In light of the problems encountered with the conventional balloon catheters discussed above, it is an object of the present invention to provide a balloon catheter equipped with a catheter shaft composed of a multi-lumen tube with a good balance of mutually conflicting characteristics, such as guide wire lubricity, pushability (the efficient transmission to the distal end of the pushing force applied to the proximal end of the catheter shaft), trackability (the ability to advance smoothly along a curved blood vessel), and a small diameter (so that the catheter can be used through even narrow blood vessels), and to provide a method for manufacturing the catheter shaft used in this balloon catheter at a high yield.

It is another object of the present invention to provide a monorail type balloon catheter that can be manufactured by forming a smooth guide wire inlet with few physical steps by a simple and stable process, and precisely joining the shafts by a simple process, regardless of the skill level of the worker.

It is a further object of the present invention to conduct comparative investigations into conventional balloon materials and manufacturing methods, find a novel balloon material, and provide a balloon catheter that solves the above problems encountered in the past and can meet the requirements of medical applications, as well as a method for manufacturing a balloon used therein. More specifically, it is an object to suppress variance in the bursting pressure of the balloon and thereby raise the rated bursting pressure set forth in FDA guidelines (that is, the guaranteed pressure resistance) even with a thin-walled balloon, and, regarding the elongation of the balloon, to precisely and easily obtain a balloon having characteristics ranging from non-compliant to semi-compliant, which is what is most required in actual medical applications, and furthermore to preserve the flexibility of the balloon and keep pinholes or winging from occurring during rewrapping.

The objects of the present invention are achieved with the features of the claims.

The present invention is a monorail type balloon catheter having a catheter shaft comprising a multi-lumen tube inclusive of a dual-lumen tube, and a balloon provided to the distal end of the catheter shaft, wherein the multi-lumen tube is equipped with at least a guide wire lumen and an inflation lumen, and is composed of a resin material having a flexural modulus that contributes to pushability and trackability, and a resin material layer having a lubricity higher than that of the constituent material of the guide wire lumen and having a surface energy of no more than 50 dyn/cm is present on the inner surface of the guide wire lumen.

The catheter shaft comprises a distal shaft provided with a balloon at its distal end portion, and a proximal shaft composed of a miscible resin material that has substantially the same melting point as said distal shaft, the distal end portion of the proximal shaft and the proximal end portion of the distal shaft are joined using a joining member composed of the same resin material as the proximal shaft or of a miscible resin material that has substantially the same melting point as the proximal shaft, and a guide wire inlet that communicates with the guide wire lumen is formed in the proximity of this joint.

Preferably, a tube composed of a highly elastic resin material having an elastic modulus higher than that of the constituent material of the inflation lumen and having a surface energy of no more than 50 dyn/cm is present in the interior of the inflation lumen.

Preferably, the outer surface of the multi-lumen tube is covered with a highly elastic resin material having an elastic modulus higher than that of the multi-lumen tube.

It is preferable if the resin material layer present on the inner surface of the guide wire lumen is formed from a tube composed of a fluororesin material or a polyolefin-based resin material inclusive of polyethylene, or is a coating layer composed of a fluororesin material.

It is also preferable if there is a circular lumen with a circular lumen cross sectional shape and a C-shaped lumen with a C-shaped lumen cross sectional shape, and the two ends in a cross section of the C-shaped lumen are

located closer to the circular lumen than the tangent to the peripheral section of the circular lumen closest to the C-shaped lumen.

It is also preferable if the multi-lumen tube is composed of a resin material having a flexural modulus of at least 2000 $kgf/cm^2$ and no more than 10,000 $kgf/cm^2$, or more specifically, composed of a resin material selected from among nylon, polyamide-based elastomers, polyesters, polyester-based elastomers, polyurethane-based elastomers, polyolefins, polyimides, polyimidoamides, and polyether imides.

In this balloon catheter, at least the distal portion of the catheter shaft may be made from a resin material and have the above-mentioned catheter shaft structure. In other words, the proximal portion of the catheter shaft may have a different constitution from the above-mentioned catheter shaft structure and resin material.

It is preferable if the highly elastic resin material of the tube provided in the interior of the inflation lumen, or the highly elastic resin material covering the outer surface of the multi-lumen tube, is a resin material with a tensile modulus of at least 1 GPa ($10^9$ Pascal), with the use of a resin material composed of a polyimide being particularly favorable.

There is also a description of methods for manufacturing the catheter shaft of the above-mentioned balloon catheter.

A method is described for manufacturing a catheter shaft, in which a tube composed of a resin material different from the resin material of which the multi-lumen tube is composed is fixed to at least one lumen out of the multi-lumen tube, wherein a multi-lumen tube having a lumen with an inside diameter that is larger than the outside diameter of this tube of a different material is prepared in advance, and the tube of a different material is inserted into the lumen in a state in which a mandrel for maintaining the inside diameter has been fitted into the center of this tube, then the multi-lumen tube is stretched by applying heat from the outside in a state in which a tensile force is applied to the multi-lumen tube in the axial direction, and the tube of a different material is fixed inside the multi-lumen tube.

It is preferable here if this mandrel is cooled with air or a liquid.

Meanwhile, a method is also described for manufacturing a catheter shaft for a balloon catheter, in which a tube composed of a resin material different from the resin material of which the multi-lumen tube is composed is fixed to at least one lumen out of the multi-lumen tube, wherein a tube composed of a different resin material and having an outside diameter that is substantially equal to or larger than the inside diameter of the lumen is prepared in advance, the tube is passed through a mold with a specific outside diameter while heat is applied from the outside, thereby forming the outside diameter of the tube to high precision, and the tube is then inserted into the lumen, one or both ends of the lumen in its axial direction are fixed to the outer peripheral surface of the tube with an adhesive agent, and the tube is fixed inside the multi-lumen tube.

It is preferable here if the tube is passed through a mold with a specific outside diameter while heat is applied from the outside in a state in which a mandrel for maintaining the inside diameter has been inserted into the center of the tube.

It is also preferable if the mold is equipped with means for blowing hot air at the tube, and it is preferable for a UV curing type, urethane-based, or cyanoacrylate-based adhesive agent to be used as the adhesive agent.

The present invention is a balloon catheter with a balloon provided to the distal end of a catheter shaft, in which at least the distal portion of the catheter shaft is composed of a resin material having a flexural modulus that contributes to pushability and trackability, wherein the balloon is preferably composed of a thermoplastic elastomer with a tensile strength of at least 300 $kgf/cm^2$ (by the method in ASTM-D638), an elongation of no more than 600% (by the method in ASTM-D638), and a Shore hardness of at least 50D (D scale), and the soft segment of the thermoplastic elastomer includes a polyester component.

It is preferable if the balloon has a rated bursting pressure of at least 12 atm and no more than 18 atm when its inflated outside diameter is 3.5 mm or less and its wall thickness is no more than 20 $\mu$m. It is also preferable if the main component of the hard segment of the thermoplastic elastomer is one or more types selected from the group consisting of polyesters, polyamides, and polyurethanes.

Furthermore, it is preferable if the flexural modulus is at least 2000 $kgf/cm^2$ and no more than 10,000 $kgf/cm^2$.

Also, the balloon catheter pertaining to the present invention may combine a catheter shaft pertaining to one of the above embodiments with a balloon pertaining to the above embodiment.

The method for manufacturing the above balloon (the seventh invention) involves the use of a thermoplastic elastomer containing a soft segment whose main component is polyester and which has a tensile strength of at least 300 $kgf/cm^2$ (by the method in ASTM-D638), an elongation of no more than 600%(by the method in ASTM-D638), and a Shore hardness of at least 50D (D scale), and comprises a first stretching step in which a balloon parison is axially stretched at least two times its length in an environment between room temperature and up to 80% of the thermal deformation temperature of said thermoplastic elastomer, and a second stretching step in which the balloon parison is radially stretched in a plurality of stages by a pressurized gas or liquid, with the elongation per stage adjusted to within a range of at least 1.2 times and no more than 2.5 times.

In the above manufacturing method, it is preferable if the balloon has a rated bursting pressure of at least 12

atm and no more than 18 atm when its inflated outside diameter is 3.5 mm or less and its wall thickness is no more than 20 μm. It is also preferable if the main component of the hard segment of the thermoplastic elastomer is one or more types selected from the group consisting of polyesters, polyamides, and polyurethanes.

It is preferable if the joining member is a cylindrical or ribbon-shaped member having an inside diameter that is larger than the outside diameter of the catheter shaft.

It is also preferable if the proximal shaft comprises a first proximal shaft that is joined to the distal shaft, and a second proximal shaft that is located closer to the proximal side than the first proximal shaft, has a greater overall length and a higher rigidity than the first proximal shaft, and is composed of a resin, a metal, or both.

The method for manufacturing this monorail type balloon catheter is such that the proximal end portion of a distal shaft provided with a balloon at its distal end portion is brought into contact with the distal end portion of a proximal shaft composed of a miscible resin material that has substantially the same melting point as the distal shaft, a joining member composed of the same resin material as the proximal shaft or of a miscible resin material that has substantially the same melting point as the proximal shaft is disposed at this contact portion, the joining member is thermally deformed to join the proximal shaft to the distal shaft, and a guide wire inlet that communicates with the guide wire lumen of the distal shaft is formed in the proximity of this joint.

It is preferable if the joining member is a cylindrical or ribbon-shaped member having an inside diameter that is larger than the outside diameter of the catheter shaft.

It is also preferable in the above method for manufacturing a monorail type balloon catheter if the joining member is covered with a heat-shrink tube, and the heat-shrink tube is heated to thermally deform the joining member.

Another method that can be used is such that the above heat-shrink tube is not used, and the entire periphery of the joining member is covered with a heating mold, and the joining member is heated and thermally deformed by the heating mold.

Fig. 1 is an overall side view of a balloon catheter;

Fig. 2 (a) is a partial vertical cross section illustrating the main components of an over-the-wire balloon catheter, , and Fig. 2 (b) is a cross section along the X1-X1 line in (a);

Fig. 3 (a) is a partial vertical cross section illustrating an embodiment of the balloon catheter pertaining to the present invention, and Fig. 3 (b) is a cross section along the X2-X2 line in (a);

Fig. 4 (a) is a partial vertical cross section illustrating another embodiment of the balloon catheter pertaining to the present invention, Fig. 4 (b) is a cross section along the X3-X3 line in (a), and Fig. 4(c) is a cross section along the X4-X4 line in (a);

Fig. 5 (a) is a partial vertical cross section illustrating the main components of an over-the-wire balloon catheter pertaining to an illustrative example, and Fig. 5 (b) is a cross section along the X5-X5 line in (a);

Fig. 6 (a) is a partial vertical cross section illustrating the main components of the balloon catheter pertaining to the invention, and Fig. 6 (b) is a cross section along the X6-X6 line in (a)

Fig. 7 (a) is a diagram of the first step in the method for manufacturing a catheter shaft pertaining to the present invention, and Fig. 7 (b) is a cross section along the X7-X7 line in (a);

Fig. 8 is a diagram illustrating the final state in which the multi-lumen tube has been stretched from the state shown in Figure 7, with (a) being a simplified side view of this multi-lumen tube, and (b) a cross section along the X8-X8 line in (a);

Fig. 9 is a diagram of the first step in the method for manufacturing a catheter shaft pertaining to the present invention, with (a) being a simplified side view illustrating the step of working the outer shape of the tube fixed in the multi-lumen tube, and (b) a simplified cross section schematically illustrating the state in which this tube has been bonded and fixed in the multi-lumen tube;

Fig. 10 is a simplified cross section used to describe the apparatus for dip-molding the covering layer on the outer surface of the multi-lumen tube;

Fig. 11 is a graph of the relationship between pressure and elongation for the balloon made of a thermoplastic elastomer pertaining to the invention, and for conventional PET and polyethylene balloons;

Fig. 12 (a) is a partial vertical cross section illustrating the main components of the monorail type balloon catheter pertaining to the present invention, Fig. 12 (b) is a cross section along the X9-X9 line in (a), and Fig. 12 (c) is a cross section along the X10-X10 line in (a);

Fig. 13 is an enlarged cross section of the joint between the distal shaft and proximal shaft of the monorail type balloon catheter pertaining to the present invention;

Fig. 14 is an enlarged cross section used to describe a method for joining the distal shaft and the proximal shaft of the monorail type balloon catheter pertaining to the present invention;

Fig. 15 is an enlarged cross section used to describe another method for joining the distal shaft and the proximal shaft of the monorail type balloon catheter pertaining to the present invention;

Fig. 16 is a partial vertical cross section illustrating another embodiment of the balloon catheter pertaining to the

present invention; and

Fig. 17 is an enlarged cross section of the joint between the first proximal shaft and second proximal shaft of the monorail type balloon catheter pertaining to the present invention.

The balloon catheter pertaining to the present invention will now be described in further detail through reference to the figures.

Figure 1 is an overall side view of an over- the-wire type of balloon catheter pertaining to an illustrative example. In the figure, 1 is a shaft, 2 is a balloon provided to the distal end portion of the shaft 1, and 3 is a manifold provided to the proximal end of the shaft 1. The balloon 2 and manifold 3 here have conventional structures. The shaft 1 is equipped with at least a guide wire lumen through which is passed a guide wire (not shown) for guiding the balloon catheter to a specific afflicted site, and an inflation lumen through which is introduced a pressure fluid for inflating or deflating the balloon 2. The manifold 3 is provided with ports 3A and 3B communicating with these lumens. In this example, the shaft 1 is inclusive of a dual-lumen tube having in its interior a guide wire lumen and an inflation lumen, but can also be expanded to a multi-lumen tube in which lumens for other purposes are laid out next to the above two lumens.

Figures 2 (a) and (b) illustrate an embodiment of the over-the-wire balloon catheter. Figure 2 (b) is a cross section along the X1-X1 line in (a). With this balloon catheter, a multi-lumen shaft 1A is provided over the entire length of the catheter. The basic structure of the shaft 1A comprises a multi-lumen tube 4 extrusion-molded from a resin material having a flexural modulus within a range that contributes to pushability and trackability, and on the inside of the guide wire lumen 4A thereof there is a mono- (or single-) lumen tube 5 composed of a different resin material with a high lubricity and having a circular cross sectional shape (hereinafter referred to simply as "tube 5").

The multi-lumen tube 4 is made from a resin material with a flexural modulus of at least 2000 $kgf/cm^2$ and no more than 10,000 $kgf/cm^2$. More specifically, it is made from a resin material selected from among nylon, polyamide-based elastomers, polyesters, polyester-based elastomers, polyurethane-based elastomers, polyolefins, polyimides, polyimidoamides, and polyether imides.

The tube 5 is made from a resin material that has high lubricity with respect to the guide wire and has a surface energy of no more than 50 dyn/cm. More specifically, it is made from a fluororesin material or a polyolefin-based resin material inclusive of polyethylene.

The cross sectional shape of this multi-lumen tube 4 is as shown in Figure 2 (b). In Figure 2 (b), the cross sectional shape of the guide wire lumen 4A is circular. The cross section of the inflation lumen 4B is C-shaped, and the line S1 that connects the ends 4a and 4b of this C-shaped cross section is located on the guide wire lumen 4A side of the tangent T1 to the peripheral section of the circular lumen 4A closest to the C-shaped lumen 4B. Here, a resin layer 4C that forms the tube 4 is present at the boundary between the guide wire lumen 4A and the inflation lumen 4B.

The reason that the cross sectional shape of the inflation lumen 4B is C-shaped is that this ensures the largest possible cross sectional area for the inflation lumen 4B in the portion other than the guide wire lumen 4A, which facilitates the flow of the pressure fluid through this lumen, that is, it maximizes conductance and minimizes the time it takes to inflate or deflate the balloon 2. If the time it takes to inflate or deflate the balloon 2 is shortened, this reduces the time required for dilation of a constriction in a blood vessel, or to put it another way, shortens the time that the blood vessel is blocked by the inflated balloon 2, which is advantageous in that it reduces the burden on the patient.

The interior of the tube 5 disposed in contact with the inner surface of the guide wire lumen 4A of the multi-lumen tube 4 becomes the actual guide wire lumen 4A. Also, it is important that the tube 5 be the resin material with high lubricity provided to the inner surface of this guide wire lumen 4A. In this respect, a coating layer of a fluororesin material may be formed as the resin material layer with high lubricity on the inner surface of the guide wire lumen 4A instead of the tube 5.

To provide the balloon 2 to the distal end of the shaft 1A, the distal end of the tube 5 is made to extend a specific length beyond the distal end of the multi-lumen tube 4, the distal end portion 2A of the balloon, which is in the form of a wrappable and inflatable tube, is fastened airtightly to the outer surface of the distal end portion of the tube 5, and the proximal end portion 2B of the balloon 2 is fastened airtightly to the outer surface of the distal end portion of the multi-lumen tube 4. At this point the inflation lumen 4B communicates with the interior of the balloon 2, and the distal end of the tube 5 is open all the way through the balloon 2.

Good pushability and trackability, which are an object of the present invention, will both be achieved by giving thought to the material and structure of the resin materials used so that the flexural modulus of the multi-lumen tube 4 that constitutes the shaft 1A will be within the desired range, and the lubricity of the guide wire will be enhanced by providing the tube 5 composed of a resin material with high lubricity inside the guide wire lumen 4A. Thus, it is possible to obtain a balloon catheter that strikes a good balance between the mutually conflicting characteristics of guide wire lubricity, pushability, and trackability.

An embodiment of the balloon catheter pertaining to the invention is illustrated in Figures 3 (a) and (b). Figure 3 (b) is a cross section along the X2-X2 line in (a). This balloon catheter only has the above-mentioned shaft 1A, which is flexible, has good trackability and pushability, and has good lubricity with respect to the guide wire, at the distal portion

of the catheter, such as the portion 20 to 30 cm from the distal end, and makes use of a proximal shaft 6 made of a material with a higher elastic modulus in order to achieve the desired pushability. Because the proximal shaft 6 is coaxially connected with the shaft 1A, this balloon catheter has a structure in which there is an overall improvement in pushability and trackability. Here, the distal end portion of the proximal shaft 6 is fitted and joined to the proximal end portion of the multi-lumen tube 4 that constitutes the shaft 1A, and the tube 5 that constitutes the shaft 1A extends along the entire length of both shafts inside the proximal shaft 6. The rest of the structure is the same as that of the above balloon catheter, and those members with the same structure are numbered the same and will not be described again. As a result, it is possible to achieve all of the desired characteristics that are an object of the present invention, namely, lubricity of the guide wire, better pushability and trackability, and reduced shaft diameter.

The monorail type balloon catheter pertaining to the invention will now be described through reference to Figures 4 (a), (b), and (c). Figure 4 (b) is a cross section along the X3-X3 line in (a), and (c) is a cross section along the X4-X4 line in (a). The balloon catheter in this example has a structure at the distal end portion of the catheter shaft that is flexible, has good trackability and pushability, and has good lubricity with respect to the guide wire, and that allows the guide wire to be inserted to the distal side from midway along the catheter shaft. Specifically, there is a distal shaft 1A" that is flexible and has good trackability, and a proximal shaft 1A’ that has good pushability. The proximal shaft 1A’ is fitted and joined to the end portion of the distal shaft 1A". Midway along the distal shaft 1A" is formed an opening 7 that communicates with the guide wire lumen 4A and is produced by cutting out the tube 5 and the multi-lumen tube 4 except for the inflation lumen 4B. A reinforcing wire 9 is also provided inside the catheter in order to solve a problem peculiar to a monorail type balloon catheter, namely, the structural weakening of the proximal portion of the catheter where it is made of resin and where the guide wire does not pass through. This reinforcing wire 9 is provided inside the catheter shaft such that it tapers from the proximal end portion of the catheter shaft toward the distal end portion of the inflation lumen 4B. Preferably, an opening is formed where the distal shaft and the proximal shaft have been joined by the manufacturing method discussed below (see Figures 12 to 15 and their descriptions).

The distal shaft 1A’’ and the proximal shaft 1A’ are joined by the following method. First, the distal shaft 1A", which is flexible and has good trackability, and the proximal shaft 1A’, which has good pushability, are readied. Next, the guide wire lumen 4A of the distal shaft 1A" is cut short and the interior removed, leaving behind the outer periphery of the proximal shaft 1A’. The end portion of this proximal shaft 1A’ is then fitted to the end portion of the distal shaft 1A", and the two are sealed and joined. It is preferable to use the manufacturing method discussed below (see Figures 12 to 15 and their descriptions). The rest of the structure is the same as in the above balloon catheter, and those members with the same structure are numbered the same and will not be described again. This structure results in a good balance between the characteristics of guide wire lubricity, trackability, and pushability. In the figure, a prime sign is added to the numbering of the components on the proximal side. The flow of the pressure fluid used to inflate or deflate the balloon 2 may be facilitated and the time it takes to inflate and deflate the balloon 2 may be shortened by reducing the outside diameter of the reinforcing wire 9 and increasing the cross sectional area of the inflation lumen 4B’.

An example of the over-the-wire type of balloon catheter will be described through reference to Figures 5 (a) and (b). Figure 5 (b) is a cross section along the X5-X5 line in (a). The basic structure of the catheter shaft 1B in this example comprises a multi-lumen tube 4 extrusion-molded from a resin material having a flexural modulus within a range that contributes to pushability and trackability. On the inside of the guide wire lumen 4A thereof there is a circular tube 5 composed of a different resin material with high lubricity, and on the inner surface of the inflation lumen 4B there is a tube 10 composed of a highly elastic resin material with even higher elastic modulus and pressure resistance. When a high pressure is applied to the inner surface of the inflation lumen 4B, this high pressure is received by the tube 10 with high pressure resistance, which makes it possible to greatly reduced the wall thickness of the multi-lumen tube 4. The highly elastic resin material that constitutes this tube 10 is preferably a resin material having a tensile modulus of at least 1 GPa ($10^9$ Pascal), with the use of a polyimide being particularly favorable.

In this example, a coating layer composed of a fluororesin material may be formed as the resin material layer with high lubricity on the inner surface of the guide wire lumen 4A instead of the tube 5. The rest of the structure is the same as that of the above balloon catheter, and those members with the same structure are numbered the same and will not be described again. As a result, it is possible to achieve the guide wire lubricity, shaft pushability and trackability, and reduced shaft diameter that are an object of the present invention, and to make the walls of the multi-lumen tube thinner and further reduce the outside diameter of the shaft.

An embodiment of the balloon catheter pertaining to the invention will be described through reference to Figures 6 (a) and (b). Figure 6 (b) is a cross section along the X6-X6 line in (a). The basic structure of the shaft 1C in this example comprises a multi-lumen tube 4 extrusion-molded from a resin material having a flexural modulus within a range that contributes to pushability and trackability. On the inside of the guide wire lumen 4A thereof there is a circular tube 5 composed of a different resin material with high lubricity, and on the outer surface of the multi-lumen tube 4 is formed a covering layer 11 composed of a resin material with a high elastic modulus and high pressure resistance, which allows the high pressure applied within the inflation lumen 4B to be borne by this covering layer 11 with high pressure resistance. This makes it possible to make the walls of the multi-lumen tube 4 much thinner, and thus to greatly reduce the outside

diameter of the shaft 1. The outer surface of the multi-lumen tube 4 may alternatively be covered with a covering tube composed of the same material instead of with the above covering layer 11. It is preferable for the highly elastic resin material that constitutes the covering layer 11 or covering tube to be a resin material having a tensile strength of at least 1 GPa, and more specifically to be a polyimide.

Also, a coating layer of a fluororesin material may be formed as the resin material layer with high lubricity on the inner surface of the guide wire lumen 4A instead of the tube 5 in this example. The rest of the structure is the same as that of the above balloon catheter, and those members with the same structure are numbered the same and will not be described again. As a result, it is possible to achieve all of the desired characteristics that are an object of the present invention, namely, lubricity of the guide wire, better pushability and trackability, and reduced shaft diameter.

Methods for manufacturing a catheter shaft in which the tube 5 is provided on the inner surface of the guide wire lumen as in the above examples will now be described through reference to Figures 7 (a) and (b), Figures 8 (a) and (b), and Figures 9 (a) and (b).

The first method is as follows. As shown in Figures 7 (a) and (b), first, a multi-lumen tube 4 having lumens 4A and 4B with a large inside diameter (naturally, the outside diameter of the multi-lumen tube 4 will also be larger) is fabricated, the tube 5 is inserted into the lumen 4A, and a tensile force (the force indicated by the arrow F1 in Figure 7 (a)) is applied to the ends of the multi-lumen tube 4 in the outward direction of each. If heat (such as hot air) is slowly applied by a heating apparatus 12 from one end of the multi-lumen tube 4 to the other with this tensile force still applied, the multi-lumen tube 4 will naturally stretch (be drawn), and as a result the inside diameter of the lumens 4A and 4B will shrink, with the constriction of the multi-lumen tube 4 stopping at the point when the inner peripheral surface of these lumens finally touches and is in close contact with the outer peripheral surface of the tube 5, ultimately resulting in the state shown in Figures 8 (a) and (b). A mandrel 13 is inserted ahead of time into the tube 5 inserted into the multi-lumen tube 4, which keeps the inside diameter of the tube 5 from changing during the stretching of the multi-lumen tube 4. Also, the effect of the heat applied to the tube 5 during stretching can be minimized by making the inside of the mandrel 13 hollow and allowing a coolant such as cold air to flow through this part. Instead of the above-mentioned heating apparatus 12, it is preferable to use a heating mold that covers the entire periphery of the multi-lumen tube in order to keep the outside diameter of this multi-lumen tube constant.

The second method is as follows. First, a tube 5 is fabricated from a resin material that is different from the constituent material of the lumen 4A (out of the lumens 4A and 4B of the multi-lumen tube 4, the one into which the tube 5 is inserted and fixed) and has an outside diameter that is 0 to about 0.030 mm larger than the inside diameter of this lumen 4A. Then, as shown in Figure 9 (a), in a state in which a mandrel 14 for maintaining the inside diameter has been inserted into the center of this tube 5, the tube 5 and the mandrel 14 are moved in the direction of arrow F2 while heat is applied to the tube 5 from the outside, so that the tube 5 and mandrel 14 are made to pass slowly through a mold 15 with a specific outside diameter. A heating coil 15a that heats an air flow is installed on the inside of the mold 15, and hot air 16 that has passed through this coil 15a goes through spray holes and heats the tube 5. As a result, the outside diameter of the tube 5 is precisely molded to within a range allowing easy insertion into the lumen 4A. After this, as shown in Figure 9 (b), the tube 5 is inserted into the lumen 4A, and the ends of the lumen 4A and the outer peripheral surface of this tube are fixed with adhesive agents 17a and 17b. The outer peripheral surface of the tube does not necessarily have to be fixed to both ends of the above lumen, however, and the outer peripheral surface of the tube may instead be fixed to just one end of this lumen. It is preferable to use a UV curing type of adhesive agent, a urethane-based adhesive agent, or a cyanoacrylate-based adhesive agent as the above-mentioned adhesive agent. The above-mentioned tolerance of 0 to 0.030 mm refers to the limit tolerance ($\pm$ 0.015 mm) in the extrusion molding of a small-diameter tube.

A method for forming the covering layer 11 on the outer peripheral surface of the multi-lumen tube will now be described through reference to Figure 10. This method is dip molding, featuring the use of a varnish whose main component is a polyimide. In Figure 10, 18a is a container, 18b is the varnish put into the container 18a, 18c is a die, and 18d is a coil heater. First, the above-mentioned multi-lumen tube 4 is readied, and both ends thereof are sealed off so that the varnish cannot penetrate into the lumens of this multi-lumen tube 4. This multi-lumen tube 4 is dipped in the varnish, after which it is pulled out through the die 18c, causing the varnish to adhere to the outer surface of the tube, and the tube is then passed through the heater 18d to dry and cure the varnish and form a cover film. A covering layer 19 having the specified thickness is formed by repeating this dip molding a specific number of times. In this dip molding, it is preferable from the standpoint of suppressing deformation of the multi-lumen tube 4 and tube 5 to leave the above-mentioned mandrel 13 (or 14) inside the tube. Naturally, it is a requirement that the characteristics of the multi-lumen shaft and the tubes not be affected by the curing temperature of the varnish.

The balloon catheters in the above examples strike a good balance between the mutually conflicting characteristics required of a catheter shaft, such as guide wire lubricity, pushability, and trackability, the result of which is that the balloon catheter can be smoothly guided and manipulated through curved blood vessels, curved afflicted sites, and highly constricted afflicted sites. More specifically, the following benefits (A) to (F) are achieved.

(A) Because the catheter shaft is constituted by a multi-lumen tube, and the multi-lumen tube is made from a resin material having a flexural modulus within a range that contributes to pushability and trackability, the rippling of the balloon and the loss of pushability that were problems with a catheter shaft having a coaxial structure (as in (4) of "Background Art" above) can be prevented, allowing a balloon catheter to have good trackability to be obtained.
(B) The lubricity of the guide wire is good because a resin material layer having even higher lubricity and a surface energy of no more than 50 dyn/cm is present on the inner surface of the guide wire lumen.
(C) Because the inner surface of the inflation lumen or the outer surface of the multi-lumen tube is covered with a highly elastic material that has a tensile modulus of at least 1 GPa and is able to withstand high pressure, the walls of the catheter shaft can be made thinner, which affords a reduction in shaft diameter.
(D) With the present invention there is less decrease in yield caused by the twisting of the tubes that occurs in the process of manufacturing the catheter shaft, which was a problem encountered with inventions such as that in Japanese Laid-Open Patent Application H7-132147 (as in (2) of "Background Art" above).
(E) With the present invention, the cross sectional shape of the inflation lumen is C-shaped, and the cross sectional area of this inflation lumen is made as large as possible, which allows the pressure fluid (consisting of a contrast medium or physiological saline and used for balloon inflation) to flow with less vascular resistance. As a result, the time it takes to inflate or deflate the balloon is shortened, which reduces the time required for dilation of a constriction in a blood vessel, or to put it another way, shortens the time that the blood vessel is blocked by the inflated balloon, which reduces the burden on the patient.
(F) Unlike the invention discussed in Japanese Patent 2,505,954 ((3) of "Background Art" above), the present invention does not improve the slip properties of the guide wire by reducing the bumpiness of the inner surface of the guide wire lumen, and is instead aimed at enhancing the slip properties of the guide wire by using a tube that is composed of a different material from that of the multi-lumen tube and that has the above-mentioned surface energy.

Embodiments of the structure of the balloon pertaining to the present invention and of the method for manufacturing this balloon will now be described.

The balloon pertaining to the present invention is composed of a thermoplastic elastomer with a tensile strength of at least 300 kgf/cm$^2$ (according to the method in ASTM D638) an elongation of no more than 600%, and a Shore hardness of at least 50D.This thermoplastic elastomer is made up of a hard segment with high crystallinity and a soft segment with crystallinity, and a polyester component is used for the soft segment. The balloon pertaining to the present invention is produced, for example, by blow molding using this thermoplastic elastomer. The characteristics of this balloon are greatly dependent on the hard and soft segments that make up the above thermoplastic elastomer. For instance, the crystallinity of the hard segment and the bonding strength of the hard segment and the surrounding molecules contribute considerably to the tensile strength of the balloon walls, while the structure of the soft segment, such as the length of the aliphatic sic or the polar groups, greatly affects compliance and other such properties of the balloon. Thus, materials composed of various hard and soft segments have been used as balloon materials in prior art as well.

In the elongation of a balloon during its inflation, when the inflation pressure is increased from approximately 6 atm to approximately 12 atm, an increase of 2 to 7% in the diameter of the balloon is generally defined as "non-compliant," an increase of 7 to 16% as "semi-compliant," and an increase of 16 to 40% as "compliant."

The structure of the balloon pertaining to the present invention will now be described while also describing the materials and characteristics of balloons in prior art, and the differences between the two will be pointed out.

As disclosed in Japanese Laid-Open Patent Application H3-57462, in the case of a balloon composed of just a resin with high crystallinity, such as nylon, compliance is greatly affected by the radial elongation during blow molding. Particularly when a balloon is made from a material such as nylon, characteristics ranging from compliant to non-compliant can be controlled by adjusting the radial elongation, but conversely, being able to control the characteristics over a wide range means that it is difficult to achieve precise control to the desired elongation. In particular, when a medical treatment method is selected that involves leaving a stent in a blood vessel in order to prevent re-constriction, it is most important for the elongation of the balloon to range from semi-compliant to non-compliant.

WO 90/01302 discusses a balloon in which a polyurethane-based elastomer is used in an effort to increase tensile strength and impart the desired elongation to the balloon. Japanese Laid-Open Patent Application H6-304920 discusses making a balloon by using a block copolymer material in order to increase the tensile strength and elastic stress response. In the examples given in these publications, these balloons make use of a polyurethane-based elastomer called "Pellethane" (Shore hardness: 75D or higher; made by Dow Chemical). In this polyurethane elastomer "Pellethane," however, the main component of the soft segment is a polyether, and a balloon molded by using this material and applying a high pressure of 12 atm or higher will have elongation characteristics that are compliant, even though "Pellethane" with its high hardness is used, and elongation will occur in the axial direction during the molding of the balloon. Also, when this "Pellethane" is used, there is a serious problem in that heat shrinkage tends to occur if the temperature is 60°C or higher after balloon molding.

WO 95/23619 discusses the use of a thermoplastic elastomer made up of a soft segment whose main component

is a polyether and a hard segment whose main component is a polyamide or polyester in order to create a balloon having high wall tensile strength, thin walls, and characteristics ranging from compliant to semi-compliant. The structural formula for this polyamide-based thermoplastic elastomer is as follows.

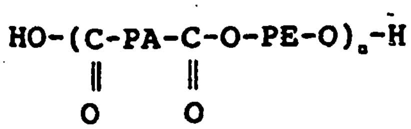

(In the formula, PA is a polyamide segment and PE is a polyether segment.)

The soft segment here is a polyether composed of a $C_2$ to $C_{10}$ diol, and more specifically is a polyether having 2 to 10 straight-chain saturated aliphatic carbon atoms between ether bonds. It is preferable to use an ether segment having 4 to 6 carbons between ether bonds, and a poly(tetramethylene ether) segment is most favorable. However, since this ether segment imparts elongation characteristics from compliant to semi-compliant, it is extremely difficult to mold the material such that a parison will have elongation characteristics close to non-compliant, and thus it is difficult to bring out consistent characteristics, that is, elongation characteristics close to non-compliant accurately and with good reproducibility.

In the prior art listed above, the block copolymers and thermoplastic elastomers discussed in WO 90 /01302. Japanese Laid-Open Patent Application H6-304920, and WO 95/23619 all have a soft segment made up of a polyether.

In contrast, with the present invention, a thermoplastic elastomer including a soft segment whose main component is a polyester is used as the material for the balloon. As a result, the elongation of the balloon can be controlled to a range from semi-compliant to non-compliant, and a balloon having merits superior to the above-mentioned prior art an be obtained.

The first merit is that variance in the bursting pressure of the balloon can be kept extremely low. If the variance in bursting pressure is low, the standard deviation (*D*) will be low, making it possible to raise the rated bursting pressure at a given average bursting pressure.

Rated bursting pressure as used here refers to the value according to FDA (Food and Drug Administration) guidelines. This rated bursting pressure guarantees that, at a statistical reliability level of at least 95%, 99.9% of the balloons will not burst at or below the lowest bursting pressure. The lowest bursting pressure is determined by the following formula.

$$\text{Lowest bursting pressure} = X - KD$$

Here, *X* is the average bursting pressure of the balloon, *D* is the standard deviation, and *K* is a constant. The constant *K* is determined using a probability *P*, reliability *C*, and number of balloons tested *n* as variables, and the relation between these variables and the constant *K* is given in a table. In the present invention, $P$ = 0.999 (99.9%). $C$ = 0.95 (95%), and n = 50, so $K$ = 3.766 is determined from the table according to FDA guidelines. The rated bursting pressure is expressed by the following formula, using the lowest bursting pressure determined by the above formula.

$$\text{Rated bursting pressure}$$
$$= \text{lowest bursting pressure} - D$$

Example 1

A balloon having an outer diameter of 3.0 mm and a thickness of 19 μm was blow molded from a tube of (outside diameter)/(inside diameter) = 0.96 mm/0.43 mm using Nubelan P4165 (trade name, made by Teijin), and this balloon was annealed at a temperature close to 95°C. Table 1 below gives data for the bursting pressure (n = 30) of the balloon (Example 1) thus produced. The resulting standard deviation is 5.65 psi (0.41 atm).

Table 1:

| Sample No. | Bursting pressure (psi) | Sample No. | Bursting pressure (psi) | Sample No. | Bursting pressure (psi) |
|---|---|---|---|---|---|
| 1 | 318 | 11 | 327 | 21 | 330 |
| 2 | 323 | 12 | 322 | 22 | 329 |
| 3 | 320 | 13 | 320 | 23 | 324 |
| 4 | 328 | 14 | 317 | 24 | 332 |
| 5 | 330 | 15 | 328 | 25 | 329 |
| 6 | 335 | 16 | 331 | 26 | 331 |
| 7 | 319 | 17 | 337 | 27 | 328 |
| 8 | 320 | 18 | 320 | 28 | 325 |
| 9 | 330 | 19 | 330 | 29 | 321 |
| 10 | 335 | 20 | 321 | 30 | 330 |

We also produced the same balloons for a number of lots, but all had a standard deviation within a range of 5.48 to 5.69 psi (0.398 to 0.412 atm), and at the same diameter had a much smaller standard deviation than polyethylene balloons (standard deviation: 1.4 atm), polyethylene terephthalate balloons (standard deviation: 1.1 atm), and nylon balloons made with nylon 12 (standard deviation: 1.0 atm). Balloons were also produced using various types of materials, whereupon the standard deviation was 0.90 atm with a polyamide-based thermoplastic elastomer composed of a soft segment of a polyether component (trade name "Pebax," trademark of Atochem), the standard deviation was 1.02 atm with a polyester-based elastomer (trade name "Hytrel," trademark of Dupont), and the standard deviation was 0.98 atm with a polyurethane-based elastomer (trade name "Pellethane," trademark of Dow Chemical). Compared to these standard deviation values, the balloon of this example is clearly superior.

The inventors also discovered that the standard deviation in the bursting pressure of a balloon can be further reduced by manipulation of the stretching step for a balloon composed of the above-mentioned thermoplastic elastomer. This stretching step involves radially stretching a balloon parison down to the final diameter with a pressurized gas or liquid in a plurality of stages, while the degree of radial stretching per stage is adjusted to within a range of 1.2 to 2.5, under an environment ranging from room temperature to a temperature that is 80% of the thermal deformation temperature of the thermoplastic elastomer. This stretching step may be conducted a number of times (three or four). This number of times becomes important when a balloon with a particularly large diameter is to be molded. The step in which the balloon parison is stretched axially may be conducted before, during, or after the radial stretching step.

Example 2

A balloon with an outside diameter of 1.8 mm was molded from a tube of (outside diameter)/(inside diameter) = 0.96 mm/0.43 mm using Nubelan P4165 (trade name, made by Teijin), after which the above-mentioned stretching step was conducted in two stages to form a balloon with a final diameter of 3.0 mm and a wall thickness of 19 $\mu$m. Then this balloon was annealed at close to 95°C (Example 2). Table 2 below gives data for the bursting pressure of this balloon (n = 30). The resulting standard deviation was 3.53 psi (0.24 atm), and it can be seen that a further reduction in standard deviation was obtained as compared to Example 1 above.

Table 2:

| Sample No. | Bursting pressure (psi) | Sample No. | Bursting pressure (psi) | Sample No. | Bursting pressure (psi) |
|---|---|---|---|---|---|
| 1 | 326 | 11 | 328 | 21 | 320 |
| 2 | 320 | 12 | 320 | 22 | 323 |
| 3 | 325 | 13 | 321 | 23 | 325 |
| 4 | 323 | 14 | 329 | 24 | 329 |

(continued)

| Sample No. | Bursting pressure (psi) | Sample No. | Bursting pressure (psi) | Sample No. | Bursting pressure (psi) |
|---|---|---|---|---|---|
| 5 | 320 | 15 | 320 | 25 | 330 |
| 6 | 330 | 16 | 320 | 26 | 330 |
| 7 | 328 | 17 | 328 | 27 | 328 |
| 8 | 321 | 18 | 322 | 28 | 322 |
| 9 | 328 | 19 | 324 | 29 | 324 |
| 10 | 326 | 20 | 324 | 30 | 326 |

The above-mentioned Nubelan series made by Teijin features an aromatic polyester for the hard segment and an aliphatic polyester for the soft segment. The Pelprene S series made by Toyobo has a similar structure. The same experiment (the same experiment as in Table 2) was conducted using this latter material, and the standard deviation in bursting pressure was 0.23 atm, which is nearly the same result as that obtained with "P4165." The chemical structure of the Pelprene series is as follows.

$$[CO\ COO(CH_2)_4O]x[(COCH_2CH_2CH_2CH_2CH_2O)m]y$$

(In the formula, x, y, and m are integers greater than or equal to 1.)

The second merit by which the balloon pertaining to the present invention is superior to the above-mentioned prior art is that its elongation characteristics can be accurately controlled to within a range from non-compliant to semi-compliant by adjusting the degree of radial stretching. Figure 11 shows the results of measuring the elongation characteristics for polyethylene, PET (polyethylene terephthalate), and Nubelan "P4165" made by Teijin. The degree of radial stretching is given in parentheses after the sample in the graph in Figure 11.

A balloon made from Nubelan "P4165" was wrapped and then heat-set by the application of heat (70 to 80°C) for 5 to 10 minutes. This balloon was repeatedly inflated and deflated by applying a pressure of 12 atm, but the winging during rewrapping that is seen with a balloon produced from polyethylene terephthalate was not observed. This is because a thermoplastic elastomer containing a soft segment whose main component is a polyester has a lower crystallinity than polyethylene terephthalate, so the flexibility needed for a balloon can be obtained. Also, the wall tensile strength of this balloon was not was high as that of a balloon made from polyethylene terephthalate, and a wall thickness of at least 10 μm was required as a matter of course, so no pinholes were observed, either.

With a balloon having the above first and second merits, even when the inflated outside diameter is 3.5 mm or less and the wall thickness is 10 to 20 μm, a rated bursting pressure of 12 to 18 atm can be ensured, and characteristics ranging from non-compliant to semi-compliant can be obtained with good reproducibility.

Therefore, with the balloon and its manufacturing method pertaining to the present invention, variance (standard deviation) in the balloon bursting pressure can be kept low, and a high value for the rated bursting pressure as defined in FDA guidelines can be obtained even with a thin-walled balloon. As to the elongation of a balloon, which is important when a stent is expanded, it is easy to manufacture a balloon having characteristics ranging from non-compliant to semi-compliant, which are the most required for medical situations. Furthermore, the molded balloon is pliant, and there are no pinholes or winging during rewrapping. so reliability is higher and the balloon is easier to manipulate.

Preferred embodiments of the catheter shaft used in a monorail type balloon catheter, and the method for manufacturing this catheter shaft, will now be described.

Figures 12 (a), (b), and (c) illustrate an example of this catheter shaft. Figure 12 (b) is a cross section along the X9-X9 line in (a), and Figure 12 (c) is a cross section along the X10-X10 line in (a). The catheter shaft comprises a distal shaft 20A and a proximal shaft 20A'. Figure 13 is an enlarged cross section of the joint 21 between the two shafts. These shafts 20A and 20A' are composed of a dual-lumen tube extrusion-molded from a resin material having a flexural modulus within a range that contributes to pushability and trackability, and are joined together using a joining member 22. The shafts 20A and 20A' and the joining member 22 are composed of the same resin materials or miscible resin materials with approximately equal melting points. A polyamide elastomer or the like can be used as the above-mentioned resin material. An inflation lumen 23B and a guide wire lumen 23A are formed on the inside of the distal shaft 20A, and a tube or coating layer (hereinafter referred to as the covering layer 24) with a circular cross section and composed of a different resin material with a high lubricity is present on the inner surface of this guide wire lumen 23A. This covering layer 24 has the same structure and material as the tube in the invention discussed above, and serves to enhance the lubricity of the guide wire. The guide wire lumen 23A communicates with a guide wire inlet 25 formed in the joint 21.

Meanwhile, an inflation lumen 23B' is formed on the inside of the proximal shaft 20A', and is connected to the left end of the inflation lumen 23B of the distal shaft 20A. Just as with the monorail type balloon catheter in the above invention, a reinforcing wire 26 is provided extending from the most proximal end of the proximal shaft 20A' to the distal end of the distal shaft 20A, and enhances pushability and prevents the breakage of the shaft by increasing the strength of the shaft at its distal portion.

The distal shaft 20A and the proximal shaft 20A' are joined by the following method. First, as shown in Figure 14, the proximal end of the distal shaft 20A is brought into contact with the distal end of the proximal shaft 20A' produced ahead of time by extrusion molding or the like. At this point, a mandrel 27 having an outside diameter that is approximately equal to the inside diameter of the guide wire lumen is inserted into this guide wire lumen in order to form the guide wire inlet 25 having a smooth external shape and to prevent any change in the shape of the covering layer 24 due to thermal deformation. Also, a mandrel 28 is inserted into the inflation lumens 23B and 23B' of the shafts 20A and 20A' in order to connect the inflation lumens 23B and 23B' more accurately. At the same time, the joint is covered with cylindrical joining members 22A and 22A' composed of the same resin material as the constituent material of these shafts 20A and 20A' or of a miscible resin material that has approximately the same melting point as this constituent material. A plurality of these cylindrical joining members 22A and 22A' with annular openings cut into them may be used on top of one another, or, as shown in Figure 15, ribbon-like joining members 22B and 22B' may be wound around the joint instead of using these joining members 22A and 22A'.

Next, the mandrel 27 and the joining members 22A and 22A' are entirely covered with a heat-shrink tube 29, heat is applied to the heat-shrink tube 29, which thermally deforms the joining members 22A and 22A', and this entire product is then cooled and the heat-shrink tube 29 removed. When the mandrel 27 is taken out, this forms a joint 21 having a smooth guide wire inlet 25 that is free of any step, as shown in Figure 13. Here, the joining members 22A and 22A' (22B and 22B') are composed of the same resin material or a miscible resin material having approximately the same melting point as the shafts, and this joining method makes it easier to fuse the shafts 20A and 20A', so the degree of joining of the shafts 20A and 20A' can be enhanced, and there is no loss of the characteristics required of the catheter shaft, such as trackability and pushability. The means for applying the heat here can be heated air, a heating mold made of glass or metal, a high frequency fusion mold that makes use of a high frequency electrical field, or the like.

It is important to use a material with a relatively large degree of shrinkage, as defined by ((diameter before shrinkage) - (diameter after shrinkage))/(diameter before shrinkage), as the above-mentioned heat-shrink tube. It is preferable for this degree of shrinkage to be approximately 25% or higher. This is because if the degree of shrinkage is less than about 25%, the shrinkage force of the heat-shrink tube will be too weak to obtain consistent results in joining strength and the molding state of the guide wire inlet. Examples of the heat-shrink tube material include polyolefins and Teflon.

The use of the above-mentioned heat-shrink tube is preferable, but heat may also be applied directly to the joining members using the above-mentioned heating mold or the like instead of the heat-shrink tube.

In general, when a monorail type balloon catheter is compared to an over-the-wire type balloon catheter, the yield is lower and the manufacturing cost higher because of the complexity of the manufacturing process. This is because of the problems mentioned above in (8) and (9) of "Background Art," that is, because a guide wire inlet must be formed so that the pressure fluid flowing through the inflation lumen will not leak from the joint between the proximal shaft and the distal shaft. However, the joining method of the present invention allows the shafts to be joined by a method that is easier and has superior reproducibility, and furthermore a smooth guide wire inlet with very little physical step can be formed by a simple and stable step, regardless of the skill level of the worker.

Example 3

In Example 3, the catheter shaft illustrated in Figure 12 was produced. The proximal shaft and distal shaft in this Example 3 were both formed by extrusion molding using a polyamide elastomer (trade name "Pebax 7233SA00," made by Elf Atochem) (outside diameter of proximal shaft: 0.88 mm, outside diameter of distal shaft: 0.91 mm). The overall length of the proximal shaft was adjusted to approximately 120 cm, and the overall length of the distal shaft to approximately 25 cm. The tube provided to the inner surface of the guide wire lumen of this distal shaft was formed by extrusion molding using a high-density polyethylene (trade name "HY540," made by Mitsubishi Chemical). A polyamide elastomer (trade name "Pebax 7233SA00," made by Elf Atochem), which is the same resin material as that of the shafts, was used for the joining members. The monorail type balloon catheter equipped with the catheter shaft of this Example 3 had good pushability, was easy to manipulate, etc.

A preferred variation example of the above catheter shaft will now be described through reference to Figures 16 and 17. In the figures, those members that are the same as above are numbered the same and will not be described again. The characteristic feature of the monorail type balloon catheter in this variation example is that the proximal shaft consists of a first proximal shaft 31A joined to the distal shaft 20A by the same method as that illustrated in Figures 14 and 15, and a second proximal shaft 31A' fitted and joined to the most proximal portion of this first proximal shaft 31A,

and the second proximal shaft 31A' has a greater overall length, is harder, and is more rigid than the first proximal shaft 31A. Figure 17 is an enlarged cross section of the joint 30 between this first proximal shaft 31A and second proximal shaft 31A'. The distal end of the second proximal shaft 31A' gradually tapers off in diameter so as to join continuously (smoothly) with the outer peripheral surface of the first proximal shaft 31A. From the standpoint of improving pushability, it is preferable for the second proximal shaft 31A' to be composed of a material with higher rigidity than that of the first proximal shaft 31A. For instance, a metal tube such as a hypodermic injection tube, or a metal tube whose outer surface is covered with a resin, can be used as this second proximal shaft 31A', or a tube composed of a resin with a high elastic modulus, such as a polyimide, can be used. The second proximal shaft 31A' composed of this material is advantageous in terms of management, in that the tolerance in the inside diameter thereof can be kept extremely low, so the tolerance in the bonding clearance between the first proximal shaft 31A and the second proximal shaft 31A' will be small.

When there is extreme variation in rigidity at the boundary between the first proximal shaft 31A and the second proximal shaft 31A', there may actually be a loss of pushability, so to prevent this, a reinforcing wire 33 is provided on the inside along both shafts 31A and 31A'.

Example 4

The catheter shaft illustrated in Figures 16 and 17 was produced as Example 4. The distal shaft and first proximal shaft of this Example 4 are the same as in Example 3 above, and were formed entirely by extrusion molding using a polyamide elastomer (trade name "Pebax 7233SA00," made by Elf Atochem) (outside diameter of proximal shaft: 0.88 mm, outside diameter of distal shaft: 0.91 mm). The overall length of the first proximal shaft was adjusted to approximately 7 cm, and the overall length of the distal shaft to approximately 25 cm. The tube provided to the inner surface of the guide wire lumen of this distal shaft was formed by extrusion molding using a high-density polyethylene (trade name "HY540," made by Mitsubishi Chemical), just as in Example 3 above.

The second proximal shaft was formed from a material with higher rigidity than that of the distal shaft and first proximal shaft, and was fitted and joined to the first proximal shaft using a cyanoacrylate-based adhesive agent (trade name "4011" and "4014," made by Loctite). A urethane-based adhesive agent or other type besides a cyanoacrylate-based adhesive agent may be used here if the tube of the bonded portion is formed flexibly, although the length of the adhesive curing time may be sacrificed. The monorail type balloon catheter equipped with the catheter shaft of this Example 4 had good pushability, was easy to manipulate, etc.

In the examples given above, both the proximal shaft and the distal shaft had a dual-lumen structure, but the present invention is not limited to this, and the structure of the proximal shaft may be changed to a single-lumen structure.

The structure of the catheter shaft pertaining to the present invention is related to a balloon catheter, but can, of course, also be applied to intravascular diagnostic catheters, including intravascular ultrasound diagnostic catheters, radiation catheters, intravascular drug injection catheters, and various atelectomy catheters.

As discussed above, the balloon catheter pertaining to the present invention, and the methods for manufacturing the catheter shaft and balloon thereof, are suited to use in the fields of in percutaneous transluminal angioplasty (PTA) or percutaneous transluminal coronary angioplasty (PTCA), in which constricted areas or obstructions such as in the coronary artery, limb arteries, the renal artery, or peripheral vessels are treated by dilation

## Claims

1. A monorail type of balloon catheter having a catheter shaft (1) comprising a multi-lumen tube (4) inclusive of a dual-lumen tube, and a balloon provided to the distal end of said catheter shaft, **characterised in that** said multi-lumen tube (4) is equipped with at least a guide wire lumen (4A, 23A) and an inflation lumen (4B, 23B), and is composed of a resin material having a flexural modulus that contributes to pushability and trackability, and a resin material layer having a lubricity higher than that of the constituent material of the guide wire lumen (4A, 23A) and having a surface energy of no more than 50 dyn/cm is present on the inner surface of said guide wire lumen, (4A, 23A)
   wherein said catheter shaft (1) comprises a distal shaft (20A, 1A") provided with said balloon (2) at its distal end portion, and a proximal shaft (20A', 1A') composed of a miscible resin material that has substantially the same melting point as said distal shaft (20A, 1A"), and
   the distal end portion of said proximal shaft (20A', 1A') and the proximal end portion of said distal shaft (20A,1A") are joined using a joining member (22A, 22A', 22) composed of the same resin material as said proximal shaft or of a miscible resin material that has substantially the same melting point as said proximal shaf (20A' 1A'), and a guide wire inlet that communicates with said guide wire lumen (4A, 23A) is formed in the proximity of this joint.

2. The balloon catheter according to Claim 1, wherein a tube composed of a highly elastic resin material having an

elastic modulus higher than that of the constituent material of said inflation lumen (4B, 23B) and having a surface energy of no more than 50 dyn/cm is present in the interior of said inflation lumen (4B, 23B).

**3.** The balloon catheter according to Claim 1 or 2, wherein the outer surface of said multi-lumen tube is covered with a highly elastic resin material having an elastic modulus higher than that of said multi-lumen tube (4).

**4.** The balloon catheter according to any of Claims 1 to 3, wherein the resin material layer present on the inner surface of said guide wire lumen (4A, 23A) is formed from a tube composed of a fluororesin material or a polyolefin-based resin material inclusive of polyethylene.

**5.** The balloon catheter according to any of Claims 1 to 3, wherein the resin material layer present on the inner surface of the guide wire lumen (4A, 23A) is a coating layer composed of a fluororesin material.

**6.** The balloon catheter according to any of Claims 1 to 5, comprising a circular lumen with a circular lumen cross sectional shape and a C-shaped lumen with a C-shaped lumen cross sectional shape, wherein the two ends in a cross section of said C-shaped lumen are located closer to the circular lumen than the tangent to the peripheral section of said circular lumen closest to the C-shaped lumen.

**7.** The balloon catheter according to any of Claims 1 to 6, wherein said multi-lumen tube (4) is composed of a resin material having a flexural modulus of at least 2000 $kgf/cm^2$ and no more than 10,000 $kgf/cm^2$.

**8.** The balloon catheter according to any of Claims 1 to 7, wherein said multi-lumen tube (4) is composed of a resin material selected from among nylon, polyamide-based elastomers, polyesters, polyester-based elastomers, polyurethane-based elastomers, polyolefins, polyimides, polyimidoamides, and polyether imides.

**9.** The balloon catheter according to any of Claims 1 to 8, wherein at least the distal portion of the catheter shaft is made from a resin material and has the catheter shaft structure according to Claims 1 to 8.

**10.** The balloon catheter according to any of Claims 2 to 9, wherein said highly elastic resin material has a tensile modulus of at least 1 GPa ($10^9$ Pascal).

**11.** The balloon catheter according to any of Claims 2 to 10, wherein said highly elastic resin material is a polyimide.

**12.** The balloon catheter according to any of Claims 1 to 11, wherein said balloon (2) is composed of a thermoplastic elastomer with a tensile strength of at least 300 $kgf/cm^2$, an elongation of no more than 600%, and a Shore hardness of at least 50D, and the soft segment of said thermoplastic elastomer includes a polyester component.

**13.** The balloon catheter according to Claim 12, wherein said balloon (2) has a rated bursting pressure of at least 12 atm and no more than 18 atm when its inflated outside diameter is 3.5 mm or less and its wall thickness is no more than 20 $\mu$m.

**14.** The balloon catheter according to Claim 12 or 13, wherein the main component of the hard segment of said thermoplastic elastomer is one or more types selected from the group consisting of polyesters, polyamides, and polyurethanes.

**15.** The balloon catheter according to Claim 1, wherein said joining member (22B, 22B') is a cylindrical or ribbon-shaped member having an inside diameter that is larger than the outside diameter of said catheter shaft (1).

**16.** The balloon catheter according to Claim 1 or 15. wherein said proximal shaft comprises a first proximal shaft (31A) that is joined to said distal shaft, and a second proximal shaft (31A') that is located closer to the proximal side than said first proximal shaft (31A), has a greater overall length and a higher rigidity than said first proximal shaft (31A), and is composed of a resin, a metal, or both.

**17.** A method for manufacturing a monorail type of catheter shaft comprising a multi-lumen tube inclusive of a dual-lumen tube,
wherein the proximal end portion of a distal shaft provided with a balloon at its distal end portion is brought into contact with the distal end portion of a proximal shaft composed of a miscible resin material that has substantially the same melting point as said distal shaft,

a joining member composed of the same resin material as said proximal shaft or of a miscible resin material that has substantially the same melting point as said proximal shaft is disposed at this contact portion,
said joining member is thermally deformed to join said proximal shaft to said distal shaft, and
a guide wire inlet that communicates with the guide wire lumen of the distal shaft is formed in the proximity of this joint.

**18.** The method for manufacturing a catheter shaft according to Claim 17, wherein a cylindrical or ribbon-shaped member having an inside diameter that is larger than the outside diameter of said catheter shaft is used as said joining member.

**19.** The method for manufacturing a catheter shaft according to Claim 17 or 18, wherein said joining member is covered with a heat-shrink tube, and said heat-shrink tube is heated to thermally deform the joining member, which joins said proximal shaft to the distal shaft, after which this heat-shrink tube is removed, and a guide wire inlet is formed in the proximity of this joint.

**20.** The method for manufacturing a catheter shaft according to Claim 17 or 18, wherein the entire periphery of the joining member is covered with a heating mold, and said joining member is heated and thermally deformed by said heating mold, which joins said proximal shaft to the distal shaft, and a guide wire inlet is formed in the proximity of this joint.

## Patentansprüche

**1.** Monorail-Ballonkatheter mit einem Katheterschaft (1) mit einer Mehrlumenröhre (4), einschließlich einer Zweilumenröhre, und einem am distalen Ende des Katheterschafts vorgesehenen Ballon, **dadurch gekennzeichnet, daß** die Mehrlumenröhre (4) mit mindestens einem Führungsdrahtlumen (4A, 23A) und einem Inflationslumen (4B, 23B) ausgestattet ist und aus einem Harzmaterial mit einem Biegemodul besteht, der zur Schiebbarkeit und Führungsgenauigkeit beiträgt, und
eine Harzmaterialschicht mit einer Lubrizität, die größer ist als die des Komponentenmaterials des Führungsdrahtlumens (4A, 23A), und mit einer Oberflächenenergie von nicht mehr als 50 dyn/cm auf der Innenfläche des Führungsdrahtlumens (4A, 23A) vorhanden ist, wobei der Katheterschaft (1) aufweist: einen distalen Schaft (20A, 1A"), der an seinem distalen Endabschnitt mit dem Ballon (2) versehen ist, und einen proximalen Schaft (20A', 1A'), der aus einem mischbaren Harzmaterial besteht, das im wesentlichen den gleichen Schmelzpunkt wie der distale Schaft (20A, 1A") hat, und
der distale Endabschnitt des proximalen Schafts (20A', 1A') und der proximale Endabschnitt des distalen Schafts (20A, 1A") unter Verwendung eines Verbindungsteils (22A, 22A', 22) verbunden sind, das aus dem gleichen Harzmaterial wie der proximale Schaft oder aus einem mischbaren Harzmaterial besteht, das im wesentlichen den gleichen Schmelzpunkt wie der proximale Schaft (20A', 1A') hat, und ein Führungsdrahteinlaß, der mit dem Führungsdrahtlumen (4A, 23A) kommunizierend verbunden ist, in der Nähe dieser Verbindungsstelle ausgebildet ist.

**2.** Ballonkatheter nach Anspruch 1, wobei eine Röhre, die aus einem hochelastischen Harzmaterial mit einem Elastizitätsmodul, der größer ist als der des Komponentenmaterials des Inflationslumens (4B, 23B), und mit einer Oberflächenenergie von nicht größer als 50 dyn/cm besteht, im Inneren des Inflationslumens (4B, 23B) vorhanden ist.

**3.** Ballonkatheter nach Anspruch 1 oder 2, wobei die Außenfläche der Mehrlumenröhre mit einem hochelastischen Harzmaterial mit einem Elastizitätsmodul überzogen ist, der größer ist als der der Mehrlumenröhre (4).

**4.** Ballonkatheter nach einem der Ansprüche 1 bis 3, wobei die auf der Innenfläche des Führungsdrahtlumens (4A, 23A) vorhandene Harzmaterialschicht aus einer Röhre ausgebildet ist, die aus einem Fluorharzmaterial oder einem polyolefinbasierten Harzmaterial, einschließlich Polyethylen, besteht.

**5.** Ballonkatheter nach einem der Ansprüche 1 bis 3, wobei die auf der Innenfläche des Führungsdrahtlumens (4A, 23A) vorhandene Harzmaterialschicht eine Beschichtungsschicht ist, die aus einem Fluorharzmaterial besteht.

**6.** Ballonkatheter nach einem der Ansprüche 1 bis 5, mit einem kreisförmigen Lumen mit einer kreisförmigen Lumenquerschnittsform und einem C-förmigen Lumen mit einer C-förmigen Lumenquerschnittsform, wobei die beiden Enden eines Querschnitts des C-förmigen Lumens sich näher an dem kreisförmigen Lumen befinden als die Tangente des Umfangsabschnitts des kreisförmigen Lumens, die dem C-förmigen Lumen am nächsten ist.

**7.** Ballonkatheter nach einem der Ansprüche 1 bis 6, wobei die Mehrlumenröhre (4) aus einem Harzmaterial mit einem

Biegemodul von mindestens 2000 $kgf/cm^2$ und nicht größer als 10 000 $kgf/cm^2$ besteht.

**8.** Ballonkatheter nach einem der Ansprüche 1 bis 7, wobei die Mehrlumenröhre (4) aus einem Harzmaterial besteht, das aus Nylon, polyamidbasierten Elastomeren, Polyestern, polyesterbasierten Elastomeren, polyurethanbasierten Elastomeren, Polyolefinen, Polyimiden, Polyimidamiden und Polyetherimiden ausgewählt ist.

**9.** Ballonkatheter nach einem der Ansprüche 1 bis 8, wobei zumindest der distale Abschnitt des Katheterschafts aus einem Harzmaterial hergestellt ist und die Katheterschaftstruktur nach Anspruch 1 bis 8 hat.

**10.** Ballonkatheter nach einem der Ansprüche 2 bis 9, wobei das hochelastische Harzmaterial einen Zugelastizitätsmodul von mindestens 1 GPa ($10^9$ Pascal) hat.

**11.** Ballonkatheter nach einem der Ansprüche 2 bis 10, wobei das hochelastische Harzmaterial ein Polyimid ist.

**12.** Ballonkatheter nach einem der Ansprüche 1 bis 11, wobei der Ballon (2) aus einem thermoplastischen Elastomer mit einer Zugfestigkeit von mindestens 300 $kgf/cm^2$, einer Dehnung von nicht mehr als 600% und einer Shore-Härte von mindestens 50D besteht und das weiche Segment des thermoplastischen Elastomers eine Polyesterkomponente aufweist.

**13.** Ballonkatheter nach Anspruch 12, wobei der Ballon (2) einen Nennberstdruck von mindestens 12 atm und nicht mehr als 18 atm hat, wenn sein Außendurchmesser im aufgeblasenen Zustand 3,5 mm oder kleiner und seine Wanddicke nicht größer als 20 $\mu$m ist.

**14.** Ballonkatheter nach Anspruch 12 oder 13, wobei die Hauptkomponente des harten Segments des thermoplastischen Elastomers ein oder mehrere Typen sind, die aus der Gruppe gewählt sind, die aus Polyestern, Polyamiden und Polyurethanen besteht.

**15.** Ballonkatheter nach Anspruch 1, wobei das Verbindungsteil (22B, 22B') ein zylindrisches oder bandförmiges Teil mit einem Innendurchmesser ist, der größer ist als der Außendurchmesser des Katheterschafts (1).

**16.** Ballonkatheter nach Anspruch 1 oder 15, wobei der proximale Schaft einen ersten proximalen Schaft (31A), der mit dem distalen Schaft verbunden ist, und einen zweiten proximalen Schaft (31A') aufweist, der sich näher an der proximalen Seite befindet als der erste proximale Schaft (31A), eine größere Gesamtlänge und eine höhere Steifigkeit hat als der erste proximale Schaft (31A) und aus einem Harz, einem Metall oder aus beidem besteht.

**17.** Verfahren zur Herstellung eines Monorail-Katheterschafts mit einer Mehrlumenröhre, einschließlich einer Zweilumenröhre,
wobei der proximale Endabschnitt eines distalen Schafts, der an seinem distalen Endabschnitt mit einem Ballon versehen ist, in Kontakt gebracht wird mit dem distalen Endabschnitt eines proximalen Schafts, der aus einem mischbaren Harzmaterial besteht, das im wesentlichen den gleichen Schmelzpunkt hat wie der distale Schaft,
ein Verbindungsteil, das aus dem gleichen Harzmaterial wie der proximale Schaft oder aus einem mischbaren Harzmaterial besteht, das im wesentlichen den gleichen Schmelzpunkt hat wie der proximale Schaft, an diesem Kontaktabschnitt angeordnet wird,
das Verbindungsteil thermisch verformt wird, um den proximalen Schaft mit dem distalen Schaft zu verbinden, und ein Führungsdrahteinlaß, der mit dem Führungsdrahtlumen des distalen Schafts kommunizierend verbunden ist, in der Nähe dieser Verbindungsstelle ausgebildet wird.

**18.** Verfahren zur Herstellung eines Katheterschafts nach Anspruch 17, wobei ein zylindrisches oder bandförmiges Teil mit einem Innendurchmesser, der größer ist als der Außendurchmesser des Katheterschafts, als das Verbindungsteil verwendet wird.

**19.** Verfahren zur Herstellung eines Katheterschafts nach Anspruch 17 oder 18, wobei das Verbindungsteil mit einem Wärmeschrumpfschlauch überzogen ist und der Wärmeschrumpfschlauch erwärmt wird, um das Verbindungsteil thermisch zu verformen, was den proximalen Schaft mit dem distalen Schaft verbindet, woraufhin dieser Wärmeschrumpfschlauch entfernt wird und ein Führungsdrahteinlaß in der Nähe dieser Verbindungsstelle ausgebildet wird.

**20.** Verfahren zur Herstellung eines Katheterschafts nach Anspruch 17 oder 18, wobei der gesamte Umfang des Verbindungsteils mit einem Heizformwerkzeug eingehüllt wird und das Verbindungsteil erwärmt und durch das Heizformwerkzeug thermisch verformt wird, was den proximalen Schaft mit dem distalen Schaft verbindet, und ein Führungsdrahteinlaß in der Nähe dieser Verbindungsstelle ausgebildet wird.

## Revendications

1. Cathéter à ballonnet de type monorail ayant une tige de cathéter (1) comprenant un tube à plusieurs lumières (4) comprenant un tube à deux lumières et un ballonnet prévu sur l'extrémité distale de ladite tige de cathéter, **caractérisé en ce que :**

    ledit tube à plusieurs lumières (4) est équipé d'au moins une lumière de fil guide (4A, 23A) et d'une lumière de gonflage (4B, 23B) et composé d'un matériau en résine ayant un module de flexion qui contribue à la poussabilité et à la traçabilité, et

    une couche de matériau en résine ayant une capacité de lubrification supérieure à celle du matériau de constitution de la lumière de fil guide (4A, 23A) et ayant une énergie de surface non supérieure à 50 dyn/cm, est présente sur la surface interne de ladite lumière de fil guide (4A, 23A),

    dans lequel ladite tige de cathéter (1) comprend une tige distale (20A, 1A") prévue avec ledit ballonnet (2) au niveau de sa partie d'extrémité distale, et une tige proximale (20A', 1A') composée d'un matériau en résine miscible qui a sensiblement le même point de fusion que ladite tige distale (20A, 1A"), et

    la partie d'extrémité distale de ladite tige proximale (20A', 1A') et la partie d'extrémité proximale de ladite tige distale (20A, 1A") sont assemblées en utilisant un élément d'assemblage (22A, 22A', 22) composé du même matériau en résine que ladite tige proximale ou d'un matériau en résine miscible qui a sensiblement le même point de fusion que ladite tige proximale (20A', 1A') et une entrée de fil guide qui communique avec ladite lumière de fil guide (4A, 23A) est formée à proximité de ce joint.

2. Cathéter à ballonnet selon la revendication 1, dans lequel un tube composé d'un matériau en résine très élastique ayant un module d'élasticité supérieur à celui du matériau de composition de ladite lumière de gonflage (4B, 23B) et ayant une énergie de surface non supérieure à 50 dyn/cm est présent à l'intérieur de ladite lumière de gonflage (4B, 23B).

3. Cathéter à ballonnet selon la revendication 1 ou 2, dans lequel la surface externe dudit tube à plusieurs lumières est recouverte avec un matériau en résine très élastique ayant un module d'élasticité supérieur à celui dudit tube à plusieurs lumières (4).

4. Cathéter à ballonnet selon l'une quelconque des revendications 1 à 3, dans lequel la couche de matériau en résine présente sur la surface interne de ladite lumière de fil guide (4A, 23A) est formée à partir d'un tube composé avec un matériau en résine fluorée ou un matériau en résine à base de polyoléfine comprenant du polyéthylène.

5. Cathéter à ballonnet selon l'une quelconque des revendications 1 à 3, dans lequel la couche de matériau en résine présente sur la surface interne de la lumière de fil guide (4A, 23A) est une couche de revêtement composée d'un matériau en résine fluorée.

6. Cathéter à ballonnet selon l'une quelconque des revendications 1 à 5, comprenant une lumière circulaire avec une forme transversale de lumière circulaire et une lumière en forme de C avec une forme transversale de lumière en forme de C, dans lequel les deux extrémités dans une section transversale de ladite lumière en forme de C sont situées plus à proximité de la lumière circulaire que la tangente par rapport à la section périphérique de ladite lumière circulaire la plus proche de la lumière en forme de C.

7. Cathéter à ballonnet selon l'une quelconque des revendications 1 à 6, dans lequel ledit tube à plusieurs lumières (4) est composé d'un matériau en résine ayant un module de flexion d'au moins 2000 $kgf/cm^2$ et non supérieur à 10000 $kgf/cm^2$.

8. Cathéter à ballonnet selon l'une quelconque des revendications 1 à 7, dans lequel ledit tube à plusieurs lumières (4) est composé d'un matériau en résine choisi parmi le nylon, les élastomères à base de polyamide, les polyesters, les élastomères à base de polyester, les élastomères à base de polyuréthane, les polyoléfines, les polyimides, les polyamides imides, et les polyétherimides.

9. Cathéter à ballonnet selon l'une quelconque des revendications 1 à 8, dans lequel au moins la partie distale de la

tige de cathéter est réalisée avec un matériau en résine et a la structure de tige de cathéter selon les revendications 1 à 8.

**10.** Cathéter à ballonnet selon l'une quelconque des revendications 2 à 9, dans lequel ledit matériau en résine très élastique a un module de traction d'au moins 1 GPa ($10^9$ Pascal).

**11.** Cathéter à ballonnet selon l'une quelconque des revendications 2 à 10, dans lequel ledit matériau en résine très élastique est un polyimide.

**12.** Cathéter à ballonnet selon l'une quelconque des revendications 1 à 11, dans lequel ledit ballonnet (2) est composé d'un élastomère thermoplastique avec une résistance à la traction d'au moins 300 $kgf/cm^2$, un allongement non supérieur à 600% et une dureté Shore d'au moins 50 D, et le segment souple dudit élastomère thermoplastique comprend un composant en polyester.

**13.** Cathéter à ballonnet selon la revendication 12, dans lequel ledit ballonnet (2) a une pression de rupture nominale d'au moins 12 atm et non supérieure à 18 atm lorsque son diamètre externe gonflé est de 3,5 mm ou moins et que son épaisseur de paroi n'est pas supérieure à 20 $\mu$m.

**14.** Cathéter à ballonnet selon la revendication 12 ou 13, dans lequel le composant principal du segment dur dudit élastomère thermoplastique est un ou plusieurs types choisis dans le groupe comprenant les polyesters, les polyamides et les polyuréthanes.

**15.** Cathéter à ballonnet selon la revendication 1, dans lequel ledit élément d'assemblage (22B, 22B') est un élément cylindrique ou en forme de ruban ayant un diamètre interne qui est supérieur au diamètre externe de ladite tige de cathéter (1).

**16.** Cathéter à ballonnet selon la revendication 1 ou 15, dans lequel ladite tige proximale comprend une première tige proximale (31 A) qui est assemblée à ladite tige distale, et une seconde tige proximale (31A') qui est positionnée plus à proximité du coté proximal que ladite première tige proximale (31A), a une longueur globale supérieure et une rigidité supérieure à ladite première tige proximale (31A), et est composée d'une résine, d'un métal ou des deux.

**17.** Procédé pour fabriquer une tige de cathéter de type monorail comprenant un tube à plusieurs lumières comprenant un tube à deux lumières,
dans lequel la partie d'extrémité proximale d'une tige distale prévue avec un ballonnet au niveau de sa partie d'extrémité distale est amenée en contact avec la partie d'extrémité distale d'une tige proximale composée d'un matériau en résine miscible qui a sensiblement le même point de fusion que ladite tige distale,
un élément d'assemblage composé du même matériau en résine que ladite tige proximale ou d'un matériau en résine miscible qui a sensiblement le même point de fusion que ladite tige proximale est disposé au niveau de cette partie de contact,
ledit élément d'assemblage est thermiquement déformé pour assembler ladite tige proximale à ladite tige distale, et une entrée de fil guide qui communique avec la lumière de fil guide de la tige distale est formée à proximité de ce joint.

**18.** Procédé pour fabriquer une tige de cathéter, selon la revendication 17, dans lequel un élément cylindrique ou en forme de ruban ayant un diamètre interne qui est supérieur au diamètre externe de ladite tige de cathéter est utilisé en tant qu'élément d'assemblage.

**19.** Procédé pour fabriquer une tige de cathéter selon la revendication 17 ou 18, dans lequel ledit élément d'assemblage est recouvert avec un tube thermorétractable, et ledit tube thermorétractable est chauffé pour déformer thermiquement l'élément d'assemblage, qui assemble ladite tige proximale à la tige distale, après quoi le tube thermorétractable est retiré, et une entrée de fil guide est formée à proximité de ce joint.

**20.** Procédé pour fabriquer une tige de cathéter selon la revendication 17 ou 18, dans lequel toute la périphérie de l'élément d'assemblage est recouverte avec un moule chauffant et ledit élément d'assemblage est chauffé et thermiquement déformé par ledit moule chauffant, qui assemble ladite tige proximale à la tige distale, et une entrée de fil guide est formée à proximité de ce joint.

Fig. 1

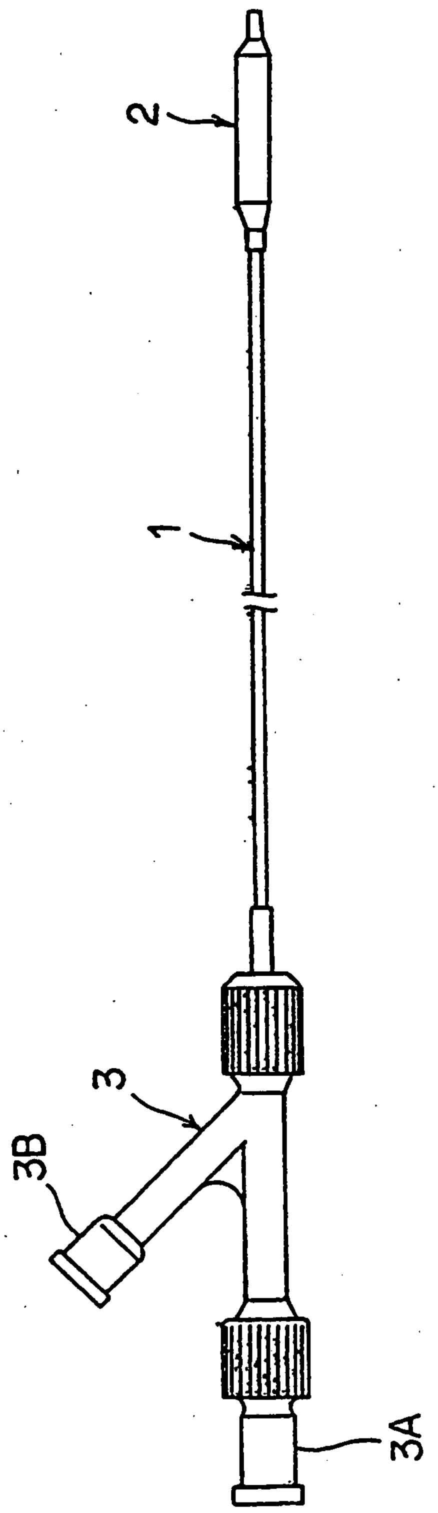

Fig. 2

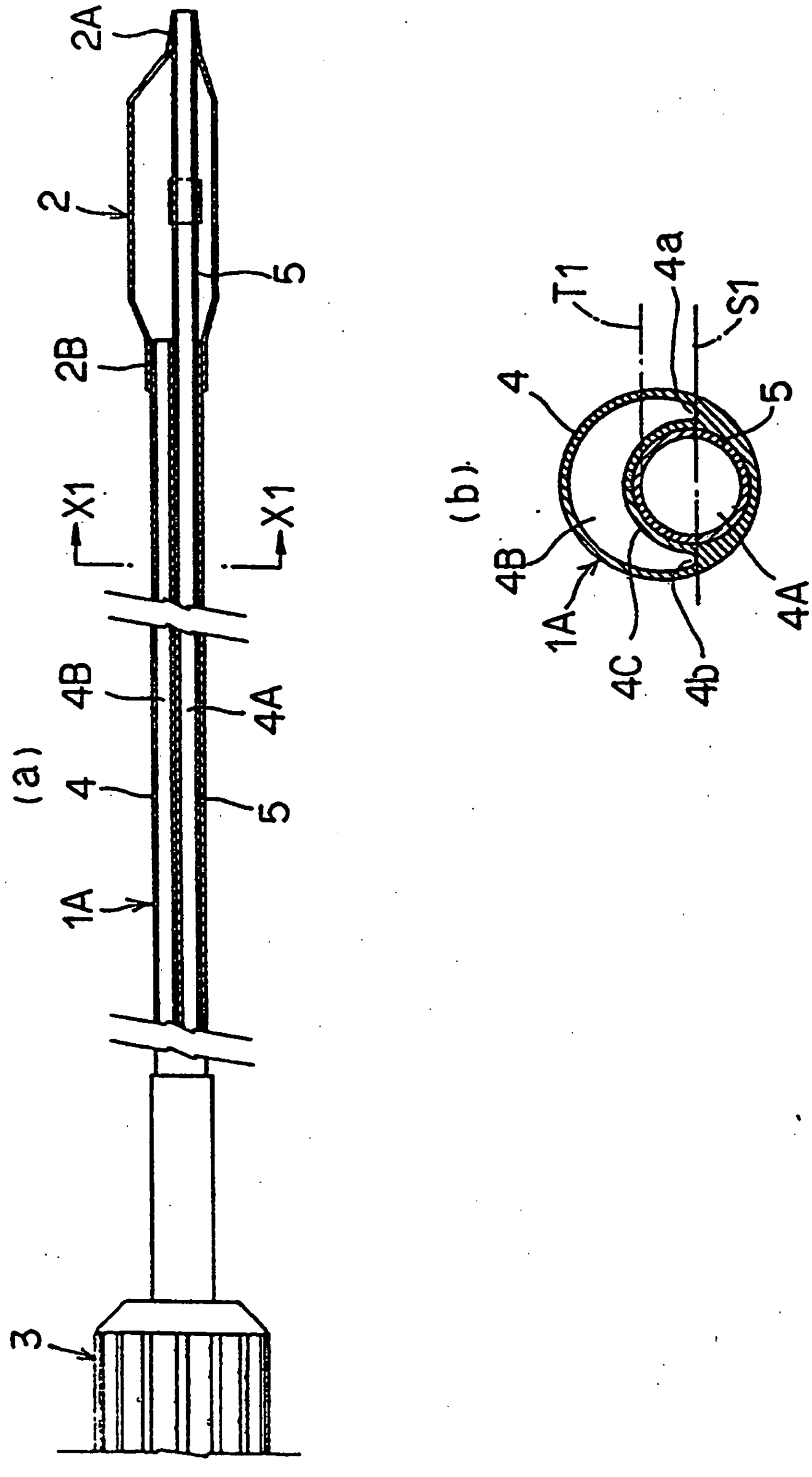

Fig. 3

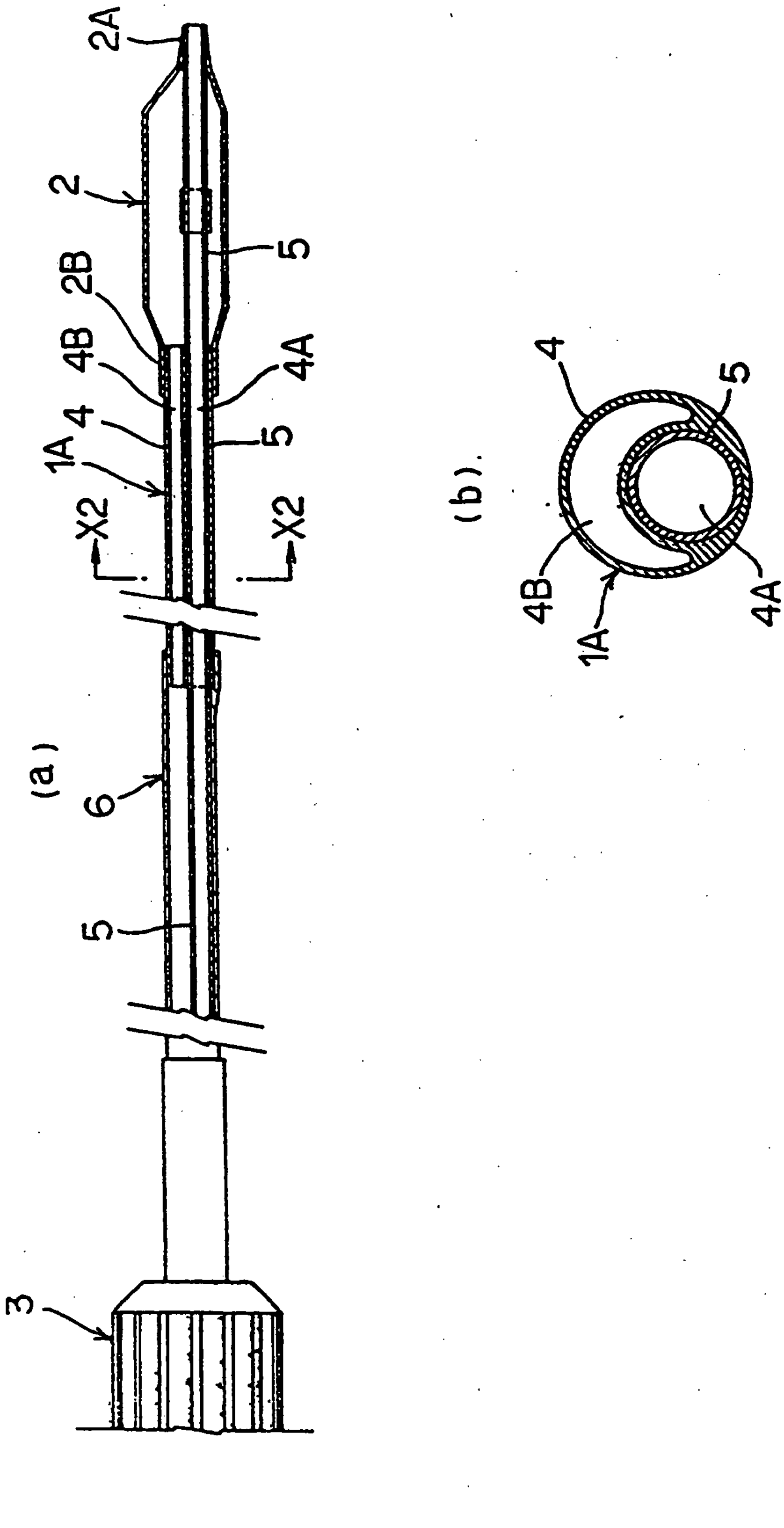

Fig. 4

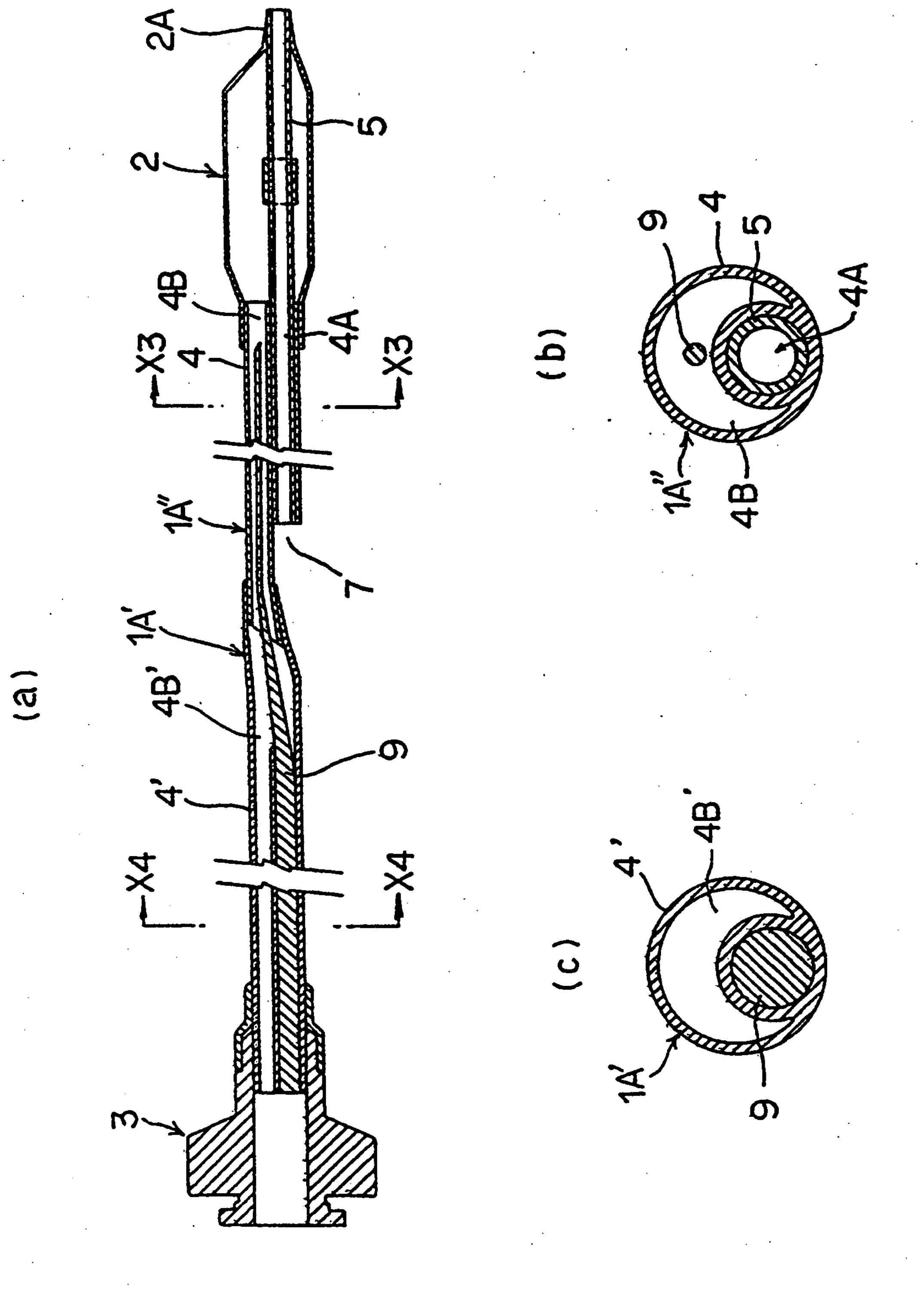

Fig. 5

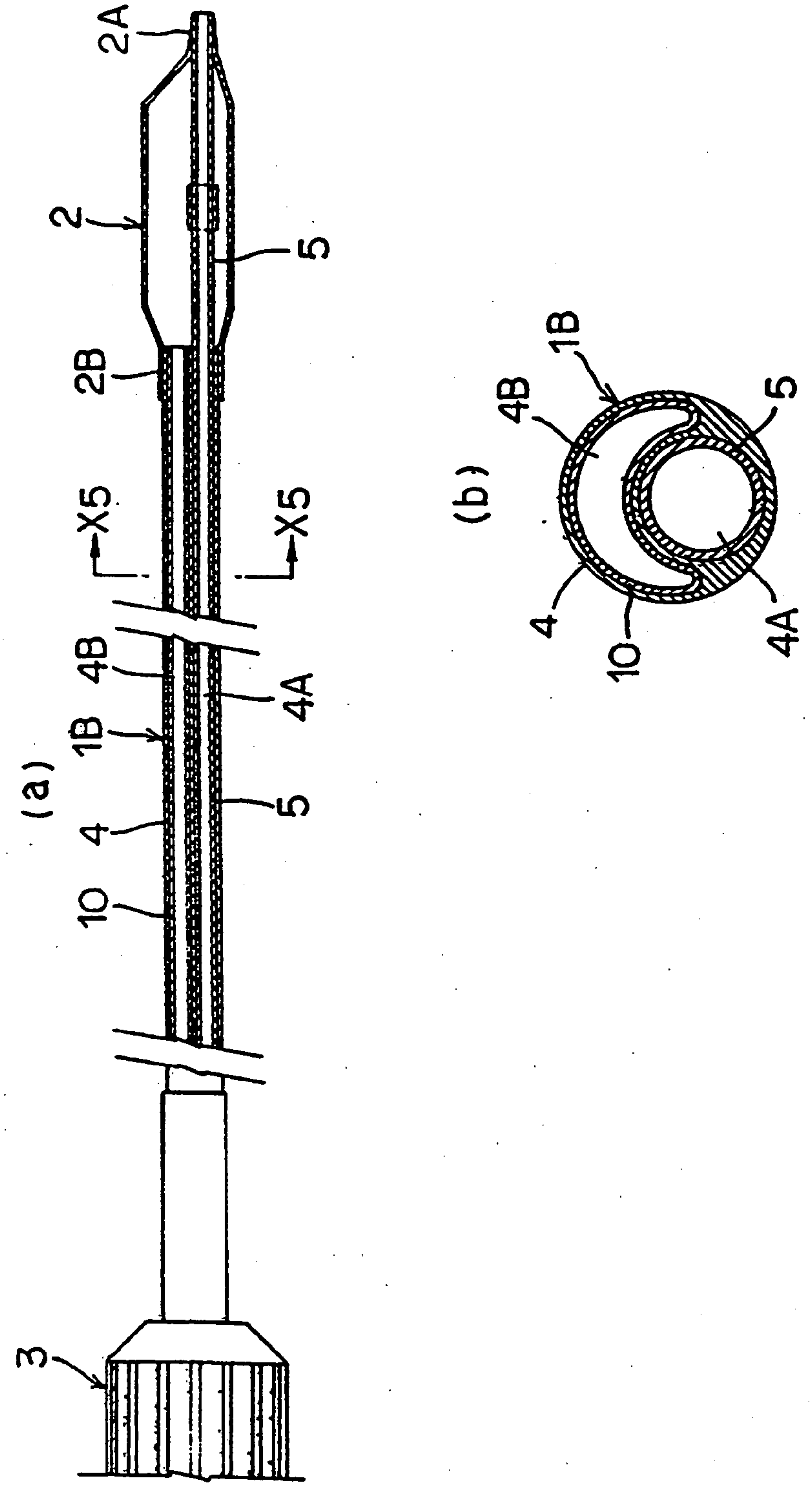

Fig. 6

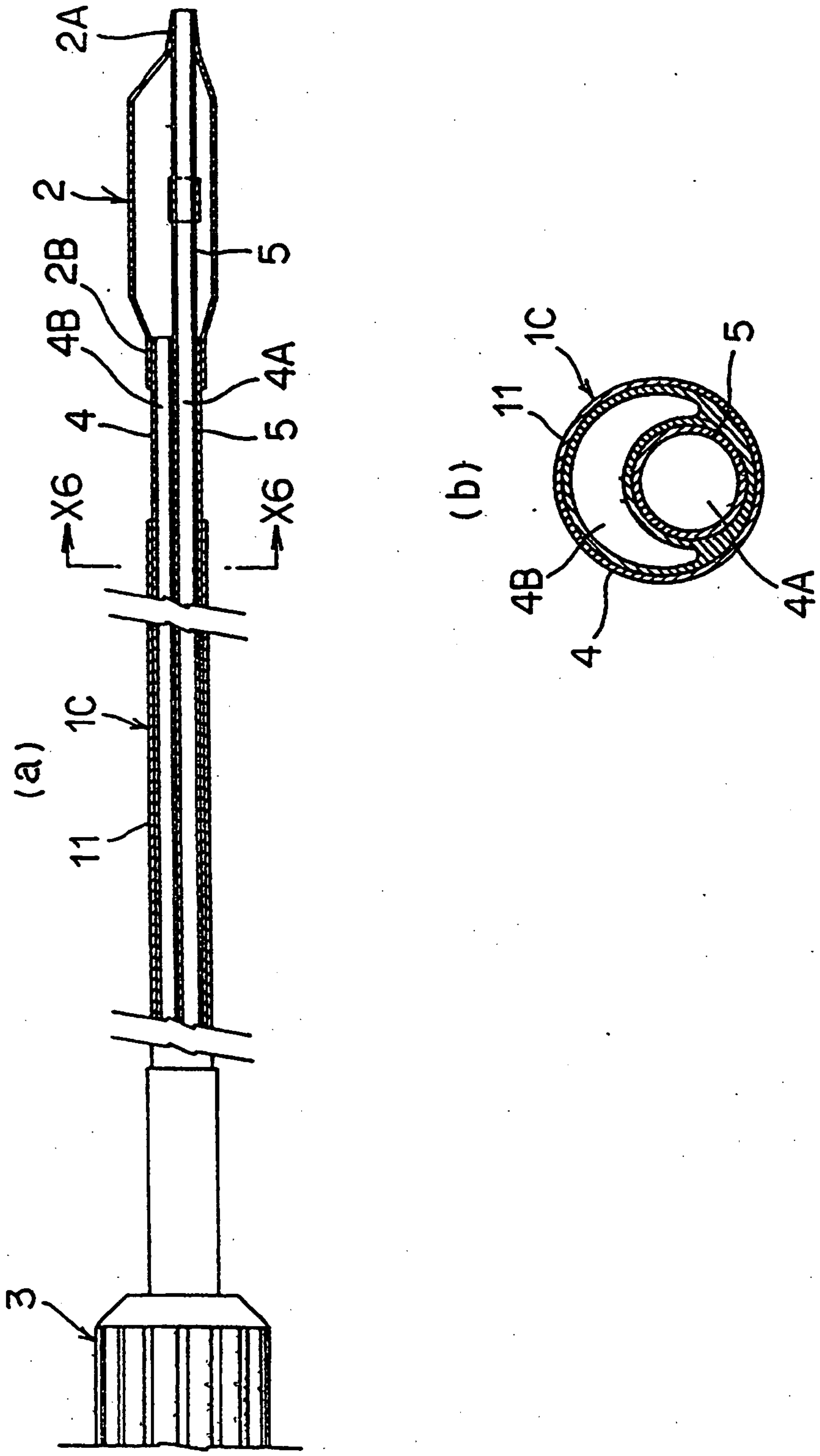

Fig. 7

(a)

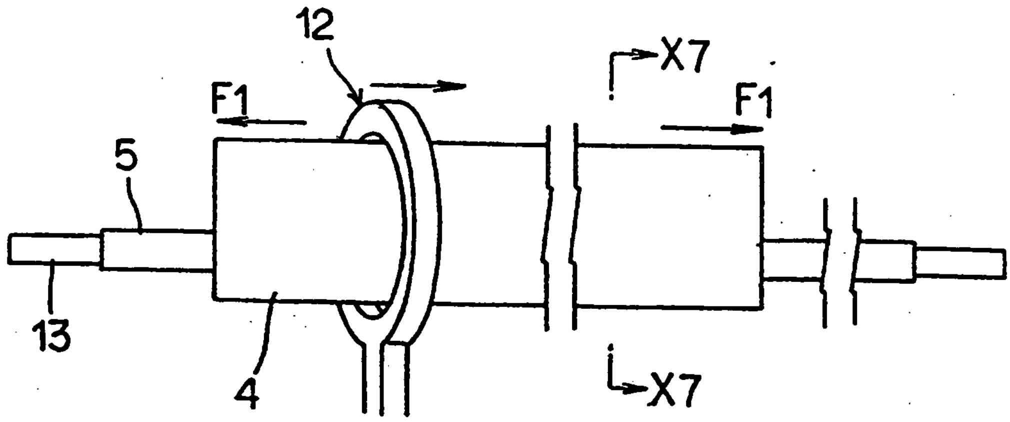


(b)

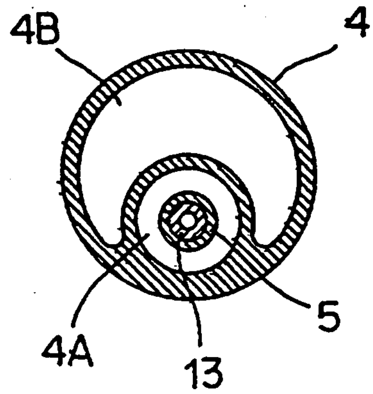

Fig. 8

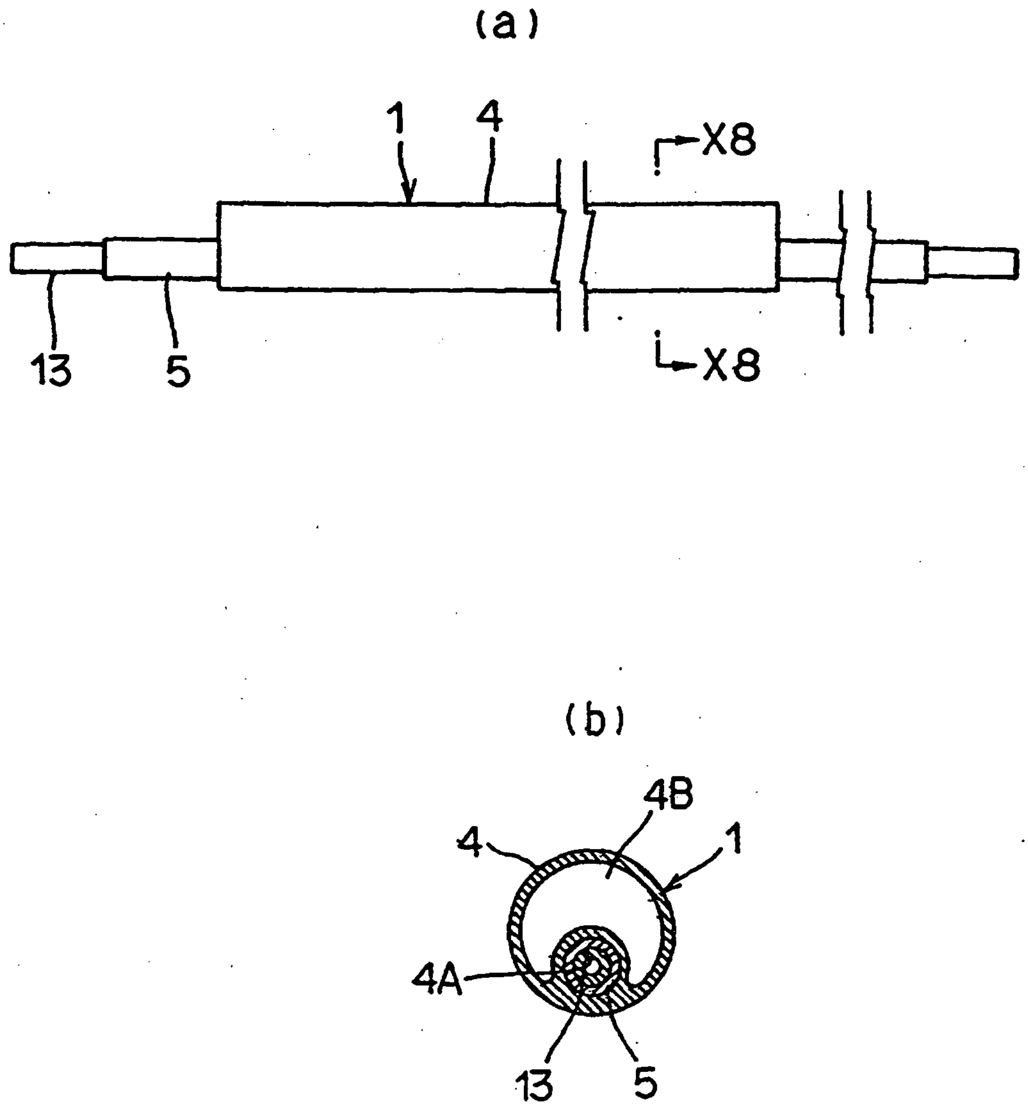

Fig. 9

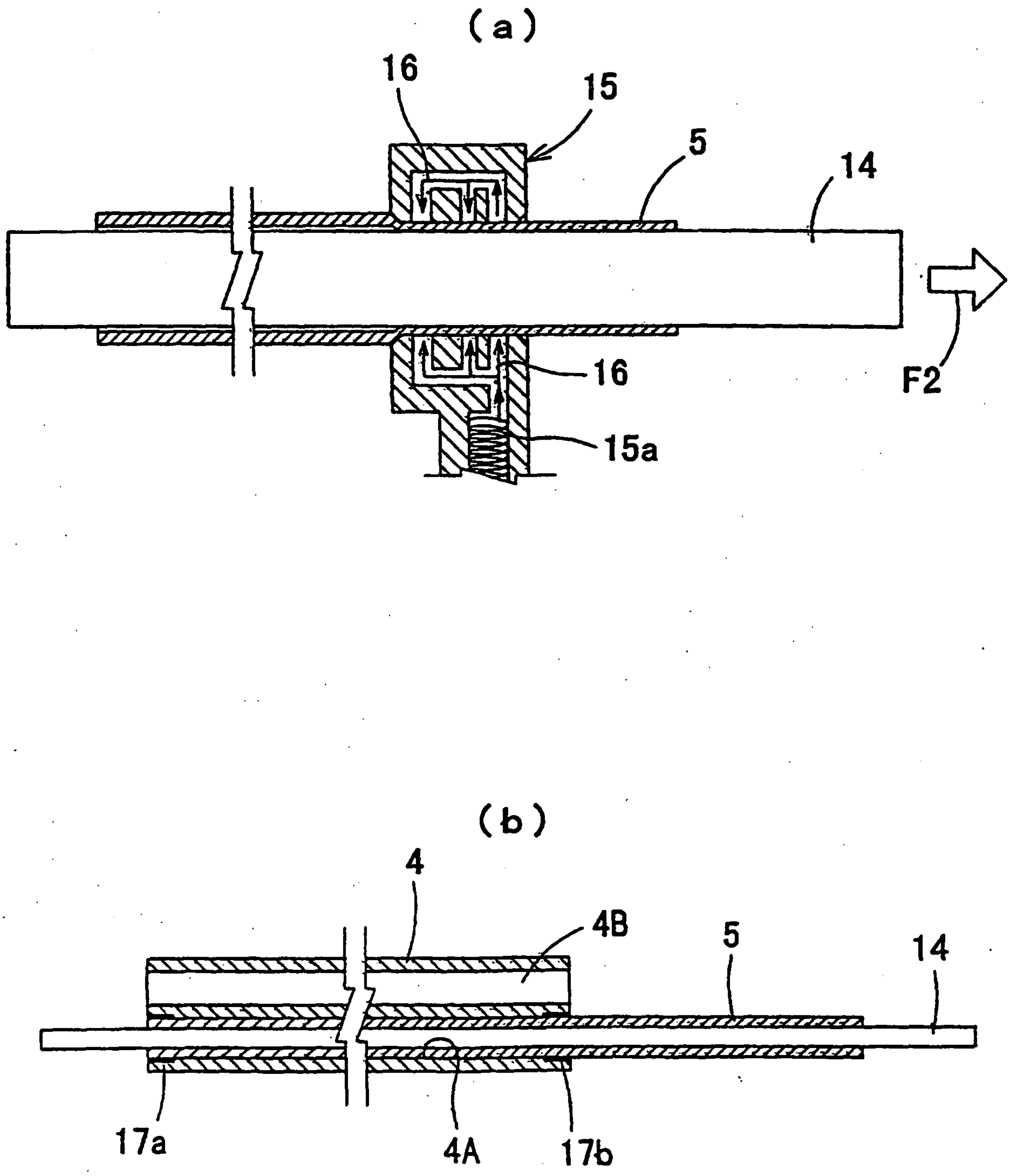

Fig. 10

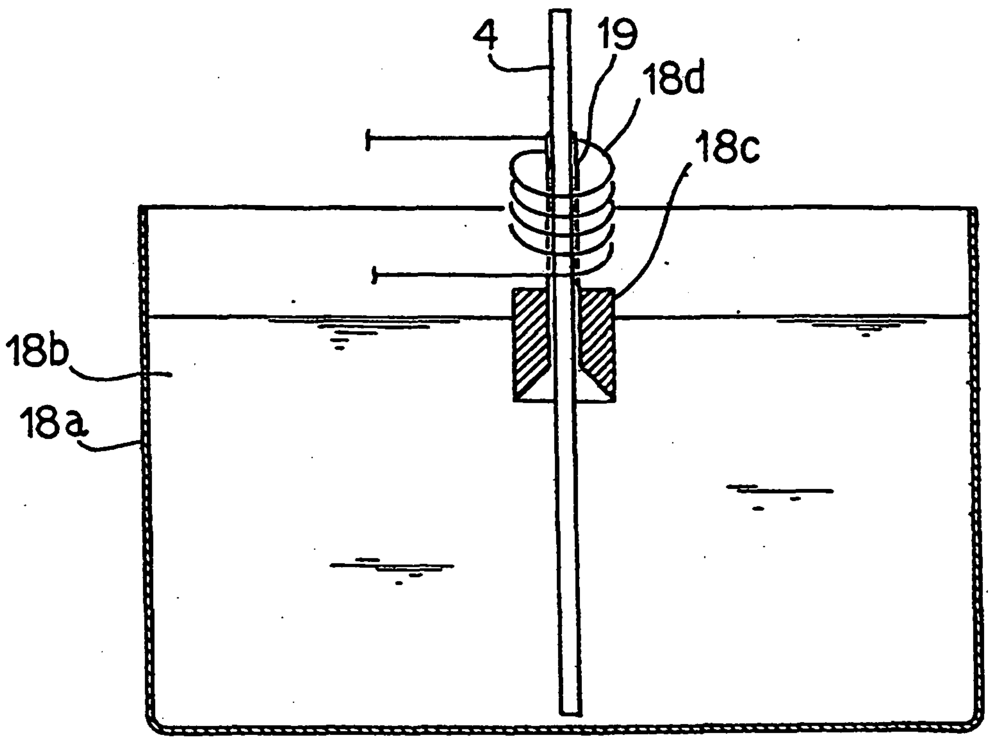

Fig. 11

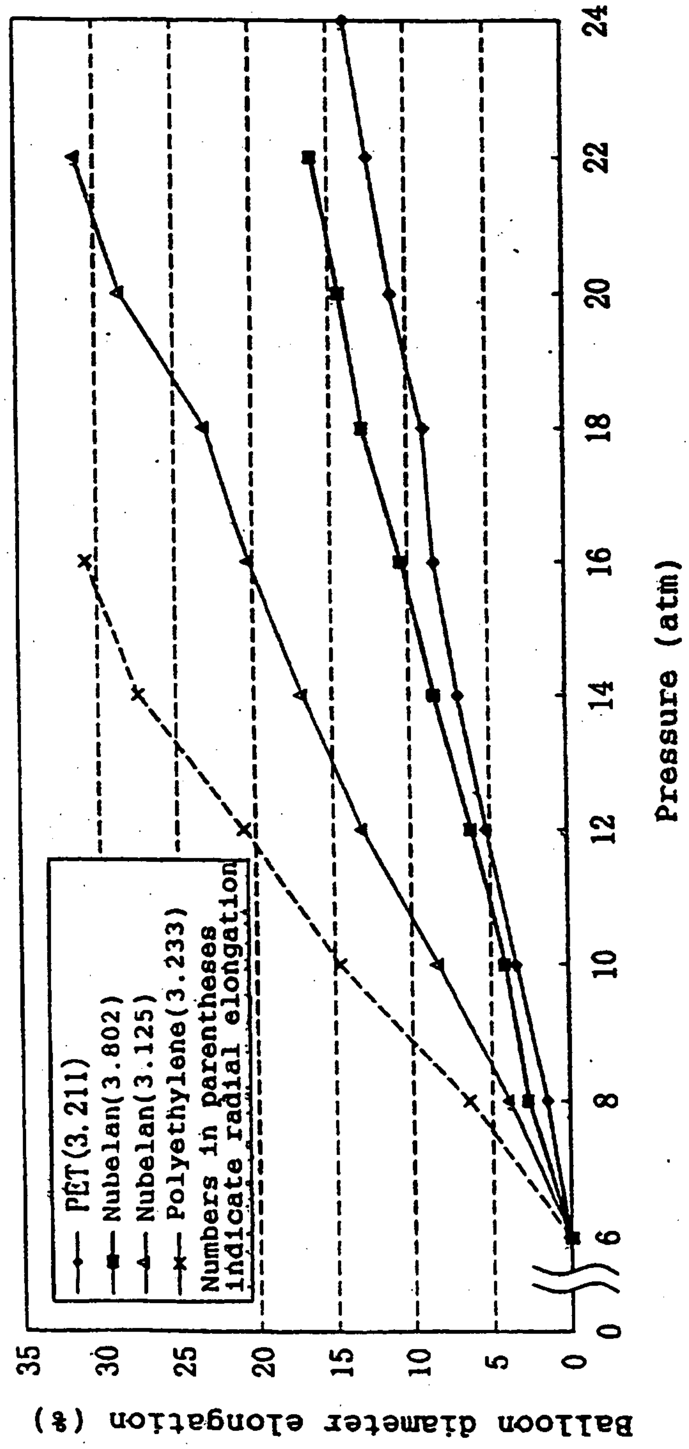
PET(3.211)
Nubelan(3.802)
Nubelan(3.125)
Polyethylene(3.233)
Numbers in parentheses indicate radial elongation
Balloon diameter elongation (%)
Pressure (atm)
0
5
10
15
20
25
30
35
0
6
8
10
12
14
16
18
20
22
24

Fig. 12

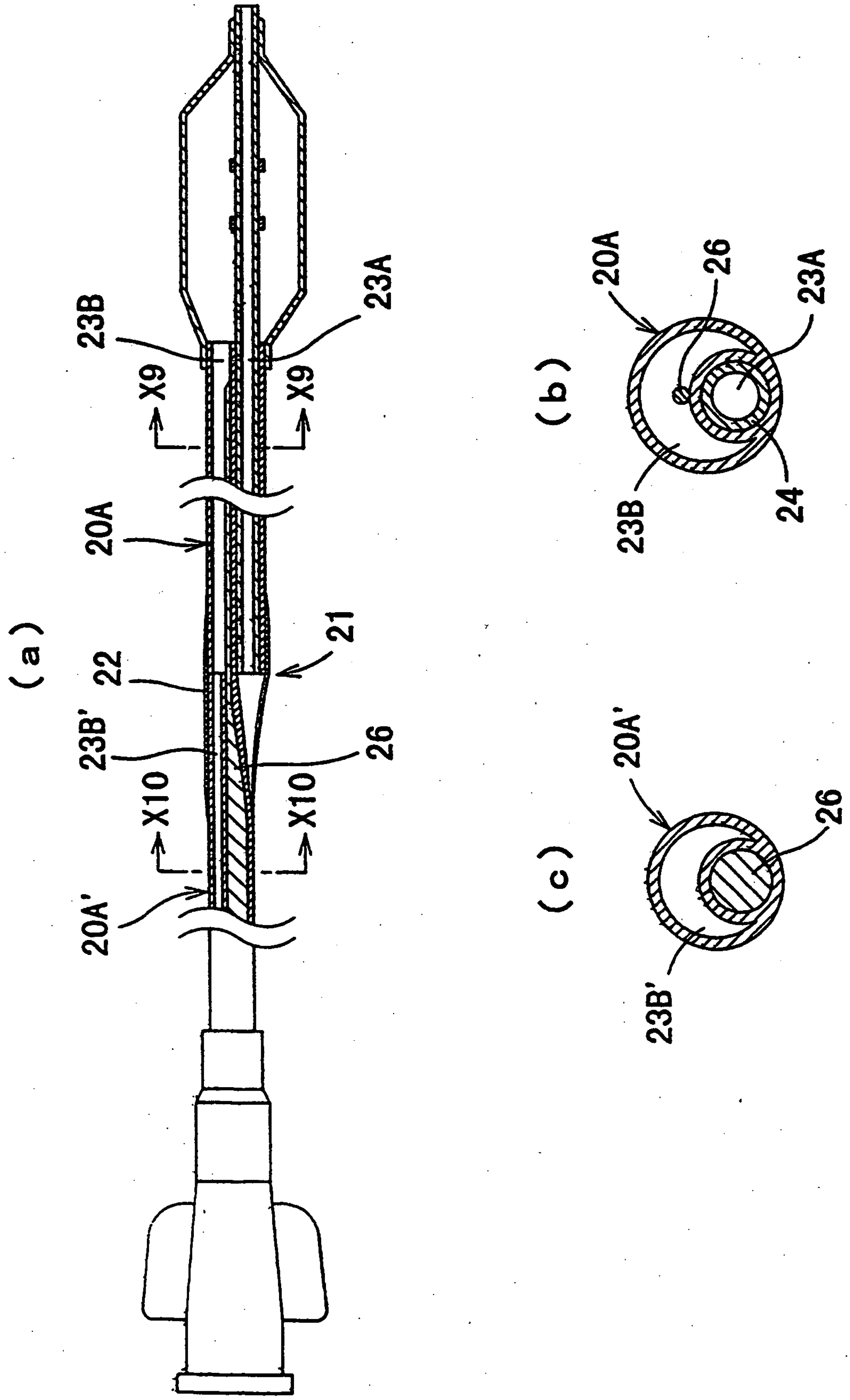
(a)
(b)
(c)
23B
23A
X9
X9
20A
21
22
23B'
26
X10
X10
20A'
20A
26
23A
23B
24
20A'
26
23B'

Fig. 13

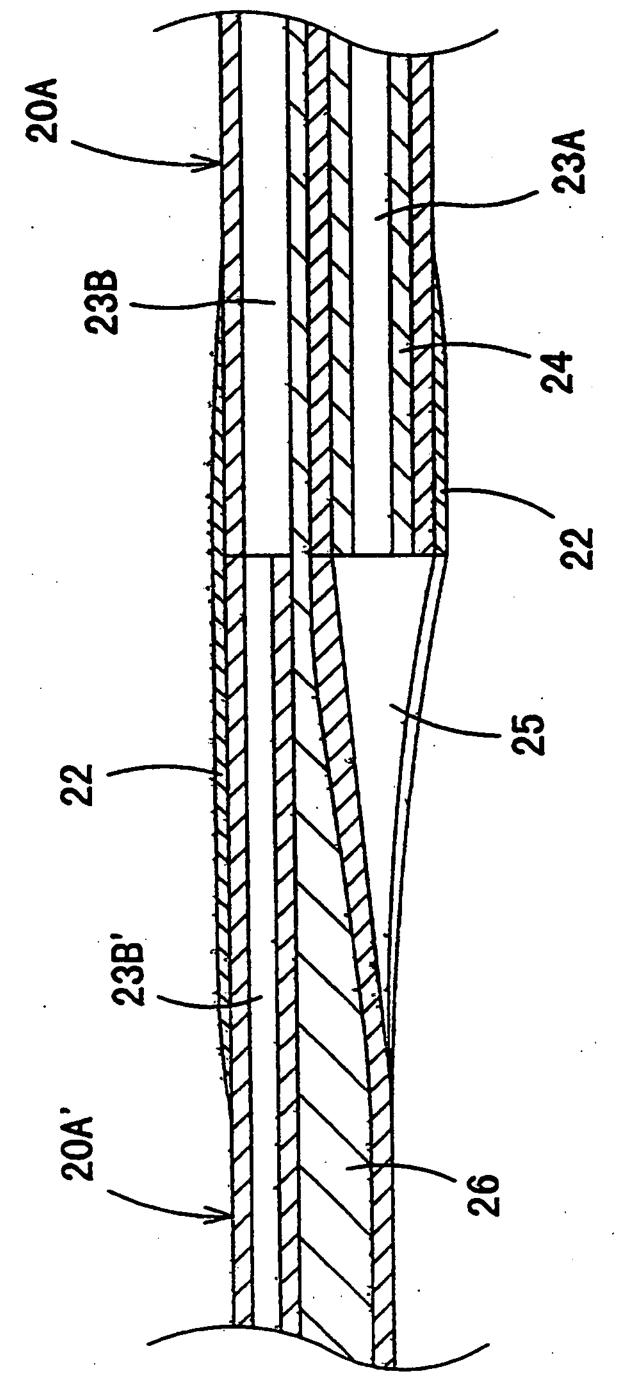

Fig. 14

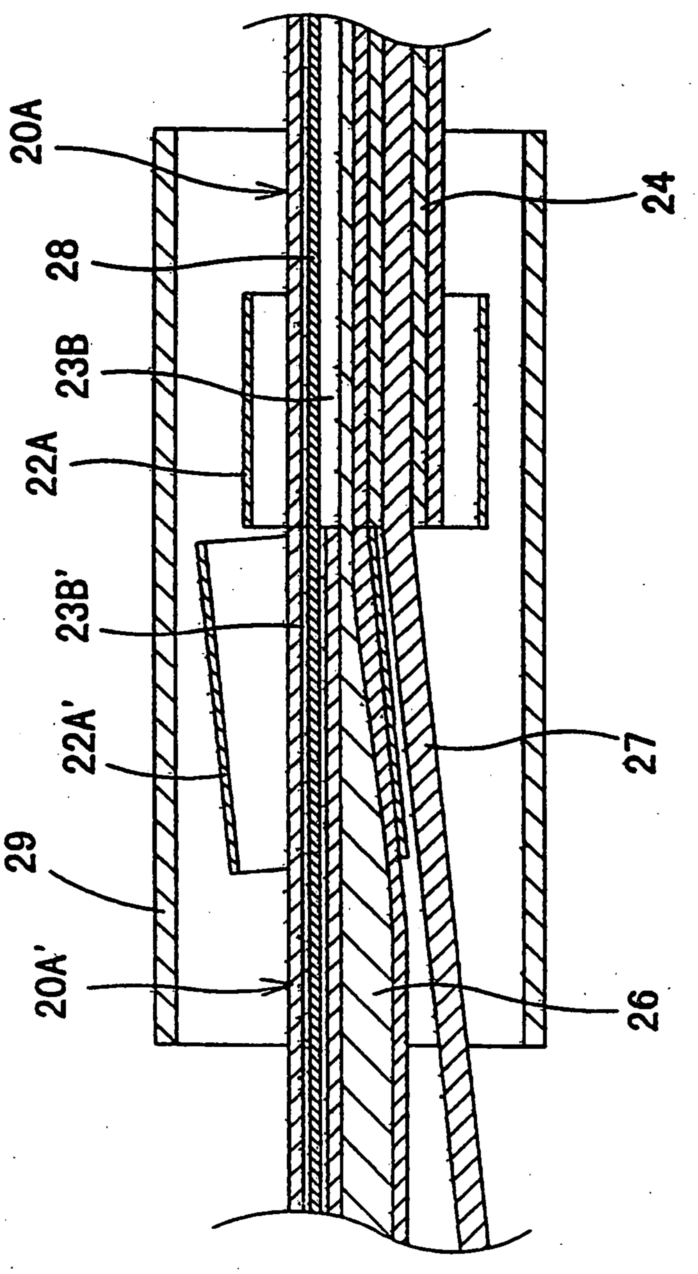

Fig. 15

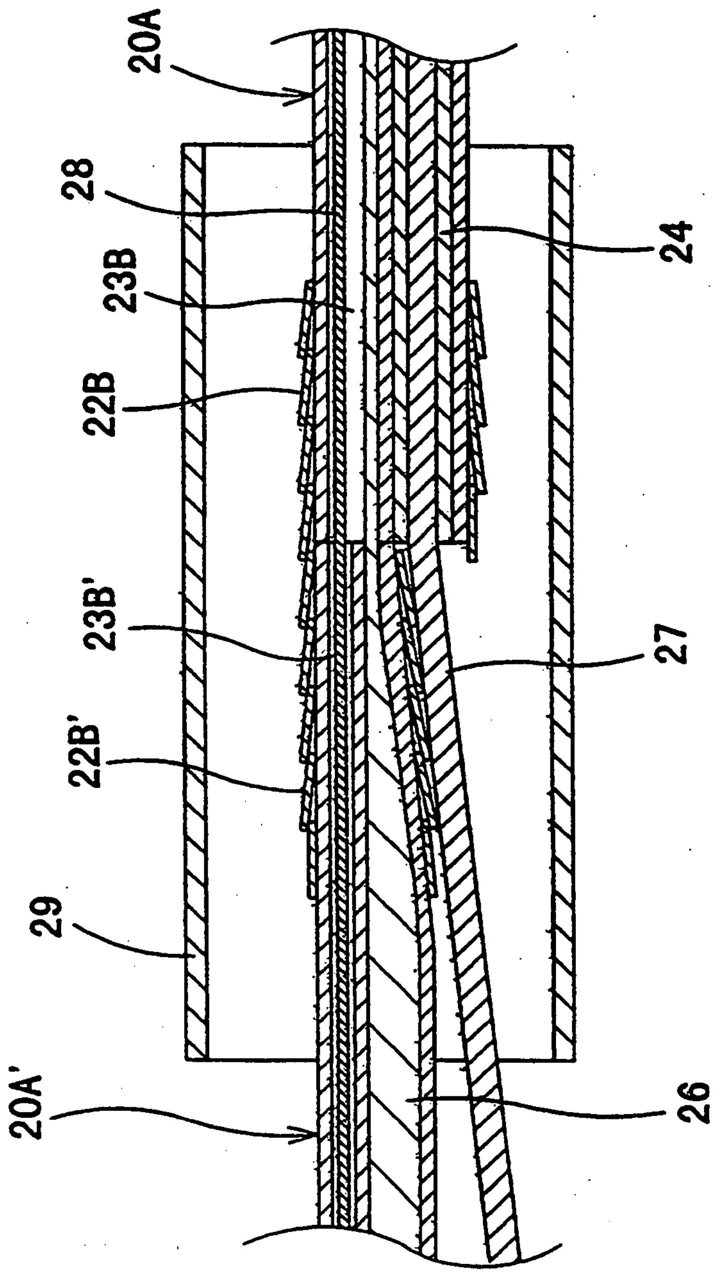

Fig. 16

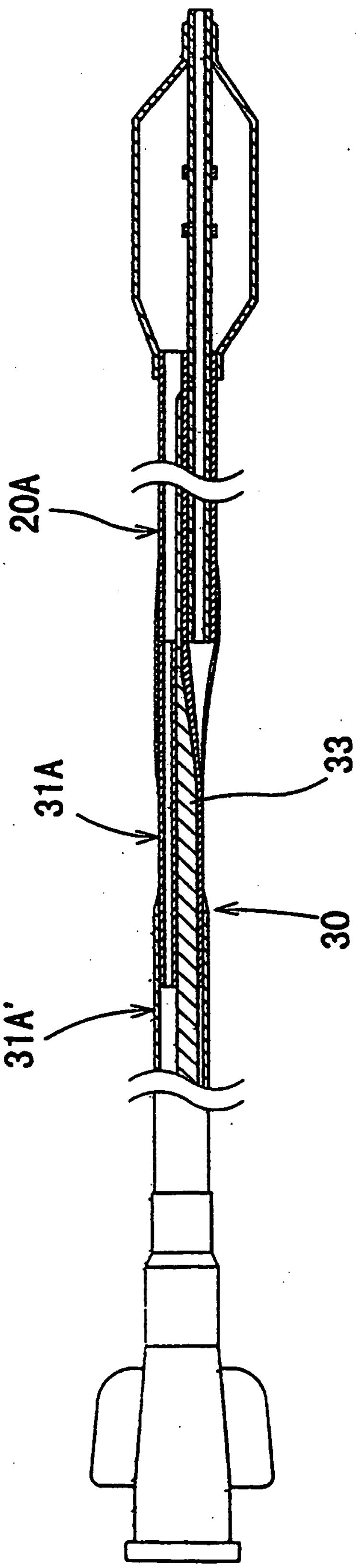

Fig. 17

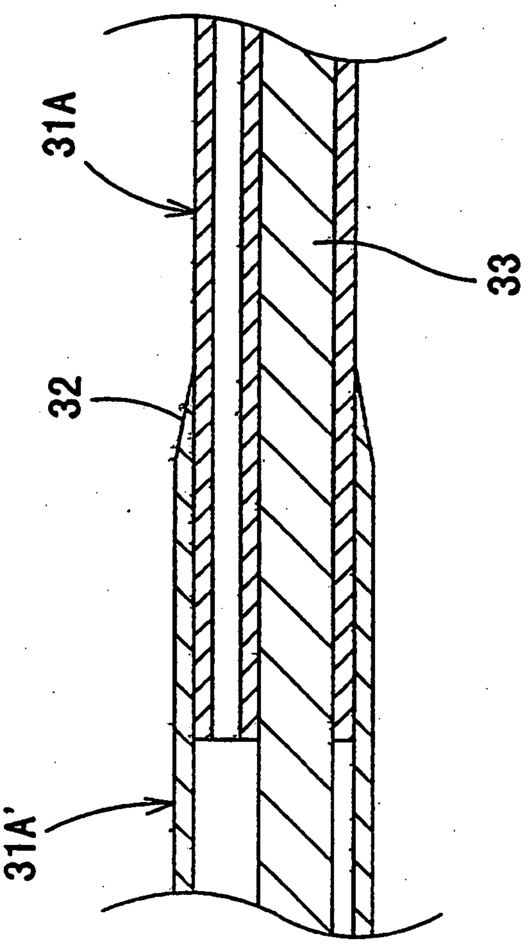

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4762129 A
- US 4748982 A
- US 5470322 A
- WO 9600099 A
- WO 9729800 A
- WO 9625970 A
- JP S63288169 B
- JP H5192410 B
- JP H7132147 B
- JP 2505954 B
- JP H2277465 B
- JP H6507105 W
- JP H6506124 W
- JP H363908 W
- JP H357462 B
- JP H6304920 B
- WO 9523619 A
- WO 9001302 A